# EUROPÄISCHE PATENTSCHRIFT

(11) **EP 1 804 735 B1**
(45) Veröffentlichungstag und Bekanntmachung des Hinweises auf die Patenterteilung: **17.10.2012**
(21) Anmeldenummer: 05802716.0
(22) Anmeldetag: 18.10.2005
(51) Int. Cl.: A61F 2/44, A61F 2/46

(54) **BANDSCHEIBENENDOPROTHESE MIT BEWEGUNGSADAPTIERTEM RAND FÜR DIE LENDEN- UND HALSWIRBELSÄULE**
INTERVERTEBRAL DISC ENDOPROSTHESIS WITH A MOTION-ADAPTED EDGE FOR THE LUMBAR VERTEBRAL COLUMN AND CERVICAL VERTEBRAL COLUMN
ENDOPROTHESE DE DISQUE INTERVERTEBRAL A BORD ADAPTE AU MOUVEMENT POUR PARTIE LOMBAIRE ET PARTIE CERVICALE DE LA COLONNE VERTEBRALE

(30) Priorität: 18.10.2004 WO PCT/DE2004/002332
(43) Veröffentlichungstag der Anmeldung: 11.07.2007
(73) Patentinhaber: Büttner-Janz, Karin, 12557 Berlin (DE)
(72) Erfinder: BÜTTNER, Eiko, 10245 Berlin (DE); BÜTTNER-JANZ, Karin, 12557 Berlin (DE)
(74) Vertreter: Tegethoff, Sebastian
(86) Internationale Anmeldenummer: PCT/DE2005/001885
(87) Internationale Veröffentlichungsnummer: WO 2006/042533

(56) Entgegenhaltungen:
- WO-A-2004/064692
- DE-A1- 3 023 353
- US-A- 6 146 421
- US-A1- 2003 135 278
- US-A1- 2004 133 278
- US-A1- 2004 199 253
- US-B1- 6 517 580

## Beschreibung

Die Erfindung betrifft eine Bandscheibenendoprothese für den vollständigen Ersatz der Bandscheibe im Lenden- und Halswirbelsäulenbereich.

Die Idee des funktionserhaltenden künstlichen Bandscheibenersatzes ist zwar jünger als der endoprothetische Ersatz der Extremitätengelenke, jedoch inzwischen fast 50 Jahre alt [Büttner-Janz, Hochschuler, McAfee (Eds.): The Artificial Disc. Springer Verlag, Berlin, Heidelberg, New York 2003]. Sie resultiert aus biomechanischen Überlegungen, nicht zufrieden stellenden Ergebnissen von Versteifungsoperationen, Erkrankungen in der Nachbarschaft von Versteifungen und aus der Entwicklung neuer Materialien mit Langzeitbeständigkeit.

Mithilfe eines funktionserhaltenden Bandscheibenimplantats ist es möglich, eine Versteifungsoperation zu umgehen, d.h. die Bewegung im Zwischenwirbelraum zu erhalten bzw. wieder herzustellen. Durch die Implantation einer künstlichen Bandscheibe gelingt es auch, im in-vitro-Experiment die biomechanischen Eigenschaften des Bewegungssegments nach einer Nukleotomie weitgehend zu normalisieren.

Unterschieden werden Implantate zum Ersatz der gesamten Bandscheibe von solchen zum Ersatz des Nucleus pulposus. Implantate zum kompletten Bandscheibenersatz sind entsprechend voluminös; sie werden von ventral oder ventrolateral eingebracht. Eine Implantation im unmittelbaren Anschluss an eine standardmäßige Nukleotomie kann mit einer Prothese zum kompletten Bandscheibenersatz demzufolge nicht durchgeführt werden.

Die Indikation zum funktionserhaltenden Bandscheibenersatz umfasst, als Alternative zur operativen Fusion, neben der primären schmerzhaften Diskopathie auch voroperierte Patienten mit einem sogenannten Postdiskotomiesyndrom, Patienten, die einen wiederholten Bandscheibenvorfall in der gleichen Etage aufweisen und Patienten, die nach einer Versteifungsoperation eine Anschlusssymptomatik in einer Nachbarbandscheibe haben.

Insgesamt werden derzeit über 10 verschiedene Prothesen zum totalen Bandscheibenersatz klinisch eingesetzt. Besonders bekannt sind bei der Lendenwirbelsäule die Charité Artificial Disc, die Prodisc, die Maverick, die FlexiCore und die Mobidisc (Übersicht in Clinica Reports, PJB Publications Ltd., Juni 2004) und bei der Halswirbelsäule die Bryan Prothese, die Prestige LP Prothese, die Prodisc-C und die PCM Prothese, welche im Folgenden beschrieben werden.

Die Prodisc Prothese für die Lendenwirbelsäule wird seit einer Weiterentwicklung zur Prodisc II seit 1999 implantiert. Es ist eine nach den Komponenten zwar 3-teilige, jedoch funktionell 2-teilige Bandscheibenprothese in der Gleitpaarung Metall-Polyethylen. Implantationen mit der Prodisc werden in der Lendenwirbelsäule und mit einem adaptierten Prothesenmodell, der Prodisc-C, auch in der Halswirbelsäule durchgeführt. Es stehen unterschiedliche Größen, Höhen (über den Polyethylenkern) und Lordosewinkel (über die Metall-Abschlussplatten) zur Verfügung. Das Vor- und Rückneigen sowie Rechts- und Linksneigen sind bei der Prothese in einem gleich großen Bewegungsumfang möglich, die Axialrotation wird konstruktionsgemäß nicht begrenzt.

Gleiches trifft zu für die beiden 2-teiligen Prothesen der Halswirbelsäule, die PCM Prothese in der Gleitpaarung Metall-Polyethylen und die Prestige LP Prothese in der Gleitpaarung Metall-Metall. Als Besonderheit weist die Prestige LP Prothese konstruktionsgemäß die Möglichkeit einer anterior-posterioren Translation auf infolge der horizontal nach ventral verlängerten Konkavität, die im Frontalschnitt den gleichen Radius hat wie die Konvexität.

Die Maverick und die FlexiCore für die Lendenwirbelsäule sind funktionell 2-teilige Prothesen mit sphärischen konvex-konkaven Gleitpartnern, beide in einer Metall-Metall-Gleitpaarung. Die Mobidisc ist dagegen eine funktionell 3-teilige Prothese in der Gleitpaarung Metall-Polyethylen mit 2 Artikulationsbereichen. Der eine Bereich ist wie bei den vorgenannten 3 Prothesen ein Ausschnitt einer Kugel mit je einer konvexen und einer konkaven Fläche der artikulierenden Partner von gleichem Radius und der andere Bereich der Mobidisc ist plan. Obwohl im planen Bereich eine Abbremsung der Axialrotation vorgesehen ist, ist diese jedoch im konvex-konkaven Artikulationsbereich nicht limitiert. Dagegen weist die FlexiCore innerhalb der sphärischen Gleitflächen über einen schmalen Bereich eines Anschlags eine Rotationsbegrenzung auf.

Als kompakte Prothese für den kompletten Bandscheibenersatz der Halswirbelsäule ist die Bryan Prothese im klinischen Einsatz, die über konvexe Titanplatten mit poröser Oberfläche an den Wirbelkörpern fixiert ist und ihre biomechanischen Eigenschaften aus einem Polyurethan-Nucleus erhält.

Die längsten Erfahrungen mit totalem Bandscheibenersatz liegen mit der Charité Prothese vor, welche Gegenstand der DE 35 29 761 C2 und der US 5,401,269 ist. Diese Prothese wurde im Jahr 1982 von Dr. Schellnack und Dr. Büttner-Janz an der Berliner Charité entwickelt und später als SB Charité Prothese benannt. 1984 erfolgte die erste Operation. Die Bandscheibenprothese wurde weiterentwickelt und seit 1987 wird der aktuelle Typ dieser Prothese, Modell III, implantiert; inzwischen weltweit über 10000mal (DE 35 29 761 C2, US 5,401,269). Die Prothese ist funktionell 3-teilig in der Gleitpaarung Metall-Polyethylen in 2 gleichen sphärischen Gleitflächen, die zum einen der transversal sich bewegende Polyethylenkern aufweist, und zum anderen die entsprechend adaptierten konkaven Pfannen in den beiden Metall-Abschlussplatten. Für die Anpassung an die Anatomie des Zwischenwirbelraums stehen in der Fläche unterschiedliche Größen der Metallplatten der Charité Prothese und verschiedene Höhen der größenadaptierten Gleitkeme sowie winklige Prothesen-Abschlussplatten zur Verfügung, die, in sagittaler Richtung umgekehrt implantiert, auch als Wirbelkörperersatz dienen können. Die Primärverankerung der Charité Prothese erfolgt über 6 Zähnchen, die sich zu dritt leicht zur Mitte versetzt neben dem vorderen und hinteren konvexen Rand jeder Prothesenplatte befinden.

Die anderen Prothesen weisen bei den wirbelkörperseitigen Metallplatten andere Primärverankerungen auf, z.B. einen Kiel, der sagittal verläuft, eine strukturierte Oberfläche, eine konvexe Form mit z.B. quer verlaufenden Rillen und Kombinationen inkl. mit unterschiedlich lokalisierten Zähnchen. Darüber hinaus können Verschraubungen zur Anwendung kommen, entweder von ventral oder von intern im Zwischenwirbelraum in den Wirbelkörper hinein.

Um die Verankerung der Prothesen-Abschlussplatten an den Wirbelkörpern langfristig zusätzlich zu gewährleisten, somit eine feste Verbindung mit dem Knochen zu erzeugen, würde, analog zu zementfreien Hüft- und Knieprothesen, eine Oberfläche geschaffen, die Chrom-Kobalt, Titan und Kalziumphosphat so miteinander verbindet, dass Knochen direkt an die Abschlussplatten heranwachsen kann. Diese Verbindung zwischen Prothese und Knochen, ohne Ausbildung von Bindegewebe, ermöglicht eine lang andauernde Fixierung der künstlichen Bandscheibe und reduziert die Gefahr von Prothesenlockerungen, Verschiebungen der Prothese und Materialbrüchen.

Ein Hauptziel beim funktionserhaltenden Bandscheibenersatz besteht darin, die Bewegungsabläufe der Prothese weitgehend dem Bewegungsmuster einer gesunden Bandscheibe anzupassen. Im unmittelbaren Zusammenhang damit steht die Bewegung und Belastung in den Wirbelbogengelenken, die bei einer Fehlbeanspruchung ein eigenes Krankheitspotential haben. Es kann zu einer Abnutzung der Wirbelbogengelenke kommen (Arthrose, Spondylarthrose), im Vollbild mit der Ausbildung von Osteophyten. Durch diese Osteophyten und auch bei einem pathologischen Bewegungsmuster der Bandscheibe allein ist die Reizung von Nervenstrukturen möglich.

Die gesunde Bandscheibe ist im Zusammenwirken mit den anderen Elementen des Bewegungssegments so aufgebaut, dass nur bestimmte Bewegungsumfänge möglich sind. So werden in der Bandscheibe zum Beispiel Vor- und Rückwärtsbewegungen des Rumpfes mit Drehbewegungen verbunden und auch Seitbewegungen kombiniert mit anderen Bewegungen ausgeführt. Die Bewegungsausschläge sind, bezogen auf die Extension (Rückneigen) und Flexion (Vorneigen) sowie das Seitneigen nach rechts und links und auch bezogen auf die Rotation, bei einer gesunden Bandscheibe im Ausmaß sehr unterschiedlich. Trotz übereinstimmender Grundmerkmale bestehen darüber hinaus Unterschiede in den Bewegungsausschlägen zwischen der Lenden- und Halswirbelsäule.

Bei Bewegungen in der Bandscheibe kommt es zu Veränderungen des Drehzentrums, d.h., die Bewegungen in der Bandscheibe erfolgen nicht um ein starres Zentrum, sondern infolge einer simultanen Translationsbewegung der benachbarten Wirbel verändert das Zentrum stetig seine Lage (inkonstantes Rotationszentrum). Die DE 30 233 53 A1 offenbart eine Zwischenwirbel-Totalprothese, insbesondere für Halswirbel, bei der zueinander gewandte benachbarte Endflächen zweier Wirbel je eine Gelenkpfanne enthalten, deren Lagerkörper mit mindestens im Wesentlichen rechteckigem oder quadratischem Grundriss verankert sind, wobei zwischen die Lagerkörper ein Abstandskörper eingelegt ist. Zur Verhinderung, dass der Abstandskörper aus den Lagerschalen herausgleiten kann, sind an den Längsseiten der Lagerkörper eine Vertiefung und dazu an der parallelen Längsseite des anderen Lagerkörpers ein vorspringender Lappen vorgesehen, wobei Vertiefung und Lappen aufeinander abgestimmt sind.

Die US 6,517,580 B1 offenbart eine Bandscheibenendoprothese, mit sphärisch geformten Artikulationsflächen. Wesentliches Element der Offenbarung der US 6,517,580 B1 ist, dass die Artikulationsfläche der Konkavität in jedem Fall größer ist, als die der Konvexität, wobei die Konkavität einen ringförmigen Rand aufweist und die Endplatte mit der Konvexität eine ringförmig Vertiefung. Die in diesem Dokument offenbarten Merkmale einer Prothese dienen der Verhinderung einer Verschiebung der Platten gegeneinander, bzw. der Zentrierung des Rotationszentrums.

In der US 6,146,421 wird eine Bandscheibenendoprothese offenbart, bei der eine Selbstzentrierung der beiden Endplatten erfolgt, welche mit benachbarten Wirbeln verbunden werden. Dies wird dadurch erreicht, dass die Endplatte, welche die Konvexität enthält, benachbart erhöhte Ränder aufweist, in welcher die Konkavität geführt wird. Diese Druckschrift offenbart keine Maßnahmen zur physiologischen Begrenzung der Bewegung benachbarter Wirbelkörper bei endgradigem Neigen.

In der US 2003/0135278 A1 wird eine Bandscheibenendoprothese offenbart, welche zum Ersatz der Bandscheibe vorgesehen ist. Die Prothese besteht aus einem oberen und einem unteren Teil, wobei zentral eine Konvexität und eine korrespondierende Konkavität angeordnet sind. Eine Prothese gemäß dieser Offenbarung erlaubt jedoch nur eine Bewegung im Bereich von 2 Grad. Nachteilig an dieser Prothese ist, dass eine Rotation um die vertikale Körperachse ungebremst stattfinden kann.

In der US2004/0133278 A1 wird eine mehrteilige Bandscheibenendoprothese offenbart. Eine Prothese gemäß dieser Offenbarung kann aus vier oder mehr Komponenten bestehen. Nachteilig an einer Bandscheibenendoprothese gemäß dieser Offenbarung ist, dass eine freie und unbegrenzte Rotation um die vertikale Körperachse erfolgen kann.

Die Offenbarung der W02004/064692 A2 beschreibt eine Bandscheibenprothese mit artikulierenden Flächen. Dabei wir zur Erzielung einer möglichst physiologischen Bewegung bei Einsatz der offenbarten Prothese ein Rand mit unterschiedlicher Neigung gezeigt. Nachteilig an der offenbarten Prothese ist, dass eine ungebremste Rotation um die vertikale Körperachse erfolgen kann und zudem bei endgradiger Neigung punktförmige Kontakte der miteinander in Kontakt kommenden Randflächen entstehen.

Die Prothese nach der DE 35 29 761 C2 zeigt hierzu einen Aufbau, der sie von anderen verfügbaren Prothesentypen unterscheidet. Diese sind wie ein Kugelgelenk aufgebaut und bewegen sich demzufolge nur um einen Drehpunkt. Durch den dreiteiligen Aufbau der Prothese nach der DE 35 29 761 C2 aus zwei metallischen Abschlussplatten und dem dazwischen liegenden, frei beweglichen Gleitkem aus Polyethylen wird der Bewegungsablauf der gesunden Bandscheibe in der humanen Wirbelsäule weitgehend nachempfunden, ausgenommen insbesondere die exakten Bewegungsausschläge in die einzelnen Bewegungsrichtungen.

Ein weiteres wichtiges Merkmal der gesunden lumbalen Bandscheiben ist deren Trapezform, die für die Lordose der Lenden- und Halswirbelsäule hauptverantwortlich ist. Die Wirbelkörper selbst sind an der Lordose nur in geringem Ausmaß beteiligt. Bei einem endoprothetischen Ersatz der Bandscheiben sollte die Lordose möglichst erhalten bleiben bzw. rekonstruiert werden. Bei der Charité Bandscheibenprothese gibt es dafür vier verschieden gewinkelte Abschlussplatten, die zudem untereinander kombiniert werden können. Jedoch bedeutet es intraoperativ einen gewissen Aufwand und die Gefahr einer Schädigung der Wirbelkörperendplatten mit erhöhter Gefahr für ein Einsinken der Prothese in die Wirbelkörper, wenn nach der Implantation der Prothese diese komplett wieder entnommen werden muss, weil eine gute Lordoseeinstellung und Belastung des Polyethylenkemzentrums nicht erzielt werden konnten.

Um ein Abgleiten bzw. Herausrutschen des mittleren Gleitpartners aus den beiden Abschlussplatten zu verhindern, ist aus der DE 35 29 761 C2 ein Gleitkem mit einer zweiseitigen teilsphärischen Oberfläche (linsenförmig) mit einem planen Führungsrand und außen mit einer Ringwulst versehen bekannt, der sich bei Extrembewegungen zwischen den beiden formadaptierten Abschlussplatten verklemmt. Auch aus der DE 102 42 329 A1 ist eine ähnliche Bandscheibenprothese bekannt, die um die Kontaktflächen herum eine Rille aufweist, in der ein mit der gegenüberliegenden Kontaktfläche in Kontakt befindlicher elastischer erster Ring zur besseren Führung eingebettet ist

Die EP 0 560 141 B1 beschreibt eine 3-teilige Bandscheibenendoprothese, welche ebenfalls aus zwei Abschlussplatten und einem dazwischen lokalisierten Prothesenkern besteht. Die in dieser Druckschrift beschriebene Bandscheibenendoprothese setzt bei Drehung ihrer Abschlussplatten in entgegengesetzte Richtungen um eine vertikale Hochachse der Rotation ohne Anschläge an den Prothesenplatten einen Widerstand entgegen. Dies wird durch ein Aufgleiten der Abschlussplatten bei der Rotation auf den Prothesenkem durch das Gewicht, welches auf die Platten infolge der biomechanischen Lastübertragung in der Wirbelsäule einwirkt, erreicht, da sich im mittigen Sagittal- und Frontalschnitt die jeweiligen Krümmungsbögen voneinander unterscheiden.

Die vorstehenden Modelle sind als Implantate dauerhaft in den Bandscheibenräumen verankert. Es kann jedoch insbesondere bei zu kleinflächiger Lastübertragung mittel- bis langfristig zu einer Migration (Verschiebung) der Abschlussplatten in die Wirbelkörper hinein und somit zur Dislokation des gesamten Implantats kommen, wodurch artifizielle Belastungen der Wirbelkörper und der umgebenden Nerven und letztendlich des gesamten Bewegungssegments auftreten können, verbunden mit erneuten Beschwerden des Patienten. Zu diskutieren sind auch die Langzeitbeständigkeit des Polyethylens und bei nicht optimaler Belastung des Polyethylens im Zwischenwirbelraum die eingeschränkte Beweglichkeit der Bandscheibenprothese. Ungenügend adaptierte Bewegungsumfänge und ungünstige biomechanische Belastungen im Bewegungssegment können unter Umständen zur Beschwerdepersistenz oder später erneut zu Beschwerden des Patienten führen.

Die US 6,706,068 B2 beschreibt hingegen eine Bandscheibenprothese, bestehend aus einem oberen und unteren Teil, wobei die Teile korrespondierend zueinander ausgebildet sind, und kein Zwischenteil als mittlerer Gleitpartner vorhanden ist. An den ineinander greifenden, miteinander artikulierenden Partnern sind unterschiedliche Formgebungen realisiert, so dass es sich um eine zweiteilige Bandscheibenprothese handelt. Diese Formgebung ist allerdings beschränkt auf Strukturen, die entweder Kanten und Ecken aufweisen, so dass auf diese Weise die beiden Prothesenteile miteinander artikulieren; in diesem Fall kann man jedoch nicht mehr von Gleitpartnern sprechen. Oder aber es sind zwei Gleitpartner gezeigt, bei denen der eine Teil konvex ausgebildet ist zur Innenseite der Prothese hin und der andere Gleitpartner entsprechend konkav ausgestaltet ist. Bei dieser Art der Prothese werden jedoch nur eingeschränkt Bewegungen der künstlichen Bandscheibe ermöglicht. Die konkave Ausstülpung entspricht dem Teil einer Kugel mit entsprechendem Krümmungsradius. Die US 6,706,068 B2 zeigt ferner eine zweiteilige Bandscheibenprothese, die auf jedem Gleitpartner konkave und konvexe Teilflächen aufweist, die mit einer entsprechenden konkaven und konvexen Teilfläche des anderen Gleitpartners korrespondieren. Hier entstehen entsprechend der Offenbarung der US 6,706,068 B2 mehrere, fixe Rotationspunkte.

Die US 5,258,031 offenbart eine zweiteilige Bandscheibenendoprothese, wobei die beiden Abschlussplatten über eine kugelige Gelenkverbindung miteinander artikulieren. Das Gelenk ist in Frontalansicht zentral in der Prothese angeordnet. In Seiten ansicht befindet sich der flächig kleine Artikulationsbereich weit außerhalb der Mitte. Die Artikulationsflächen sind im Sagittalschnitt sphärisch und im Frontalschnitt plan gestreckt, an den Enden kleinflächig teilsphärisch und anschließend plan schräg, hier jedoch kontaktfrei bei Kontakt der anderen Gelenkbereiche. Beim Seitneigen mit einer Prothese der US 5,258,031 findet daher ein Neigen über die teilsphärische Kante der Artikulationsflächen statt, bis ein Kontakt der seitlichen Innenflächen der Abschlussplatten entsteht. Die nach außen geöffneten Flächen im beidseits seitlichen Artikulationsbereich, kommen wenigstens bei einer seitlichen Bewegung nicht miteinander in Kontakt. Somit lastet bei einer seitlichen Neigung von Abschlussplatten gemäß der US 5,258,031 der auf den Platten lastende Druck zeitweilig nur auf den teilsphärischen Kanten der Artikulationsflächen. Aufgrund der punkt- bzw. kleinflächigen Druckverteilung beim Seitneigen, sind die Randbereiche der Konvexität/Konkavität einem höheren Verschleiß ausgesetzt. Die Ränder der Prothese nehmen bei den verschiedenen Bewegungen ebenfalls keinen großflächigen Kontakt miteinander auf. Sofern mit einer Prothese nach der US 5,258,031 eine Rotation in einer vertikalen Achse ermöglicht wird, ist nur noch eine streifenförmige beidseits laterale punktuelle Kontaktregion zwischen oberer und untere Abschlussplatte vorhanden.

Ausgehend von diesem Stand der Technik ist es Aufgabe der vorliegenden Erfindung, eine Bandscheibenendoprothese für den totalen Bandscheibenersatz zur Verfügung zu stellen, bei der das Bewegungsausmaß an die Anatomie und Biomechanik der Bandscheibenräume der Lenden- und Halswirbelsäule, in welche sie implantiert wird, über die Randgestaltung gezielt angepasst werden kann, so dass eine physiologische Bewegung und zugleich ein großer Kontaktbereich der Gleitpartner gewährleistet wird.

Gelöst wird diese Aufgabe durch die Merkmale der selbstständigen Ansprüche 1 und 2. Die Erfindung sieht zwei unterschiedliche Arten einer Bandscheibenendoprothese vor, nämlich eine funktionell zweiteilige und eine funktionell dreiteilige Prothese.

Eine erfindungsgemäße funktionell zweiteilige Prothese nach Anspruch 1 zeichnet sich dadurch aus, dass
a) ein erster Gleitpartner derart ausgebildet ist, dass die zur Verbindung mit einem Wirbelkörper entgegengesetzte Seite eine konvexe Krümmung (Konvexität) aufweist und die Geometrie dieser Konvexität einer Kugelkappe entspricht, wobei die Konvexität vollständig von einem Rand umschlossen wird, und
b) ein zweiter Gleitpartner auf der zur Verbindung mit einem Wirbelkörper entgegengesetzten Seite eine konkave Artikulationsfläche (Konkavität) aufweist und die Geometrie der Konkavität durch eine zur kugelförmigen Konvexität des ersten Gleitpartners korrespondierende Ausnehmung definiert ist, wobei diese vollständig von einem Rand umschlossen wird, und
c) wenigstens ein Gleitpartner (11, 12) einen zur gezielten Begrenzung der maximal möglichen Bewegung der Gleitpartner (11, 12) geeigneten Rand (18) aufweist, der aufgrund seiner variierenden Höhe Konvexität (16) oder Konkavität (17) wellenförmig umgibt, und
d) der andere Gleitpartner (11 oder 12) einen die korrespondierende Konkavität (17) oder Konvexität (16) umgebenden Rand (18) aufweist, der um die korrespondierende Konkavität (17) oder Konvexität (18) korrespondierend zum artikulierenden Gleitpartner (11 oder 12) wellenförmig verläuft oder plan ist,
e) wobei die Höhenunterschiede eines wellenförmigen Randes (18) fließend und/oder in ein oder mehreren Stufen ineinander übergehen, und
f) es bei endgradiger Neigung und/oder Rotation der Gleitpartner (11, 12) zueinander zu einer definierten Begrenzung der maximal möglichen Bewegung infolge Lückenschlusses der Ränder (18) kommt.

Die funktionell dreiteilige erfindungsgemäße Bandscheibenendoprothese nach Anspruch 2 zeichnet sich dadurch aus, dass
a) der mittlerer Gleitpartner auf Ober- und Unterseite konvexe Krümmungen (Konvexitäten) aufweist und die Geometrie dieser Konvexitäten jeweils einer Kugelkappe entspricht, und
b) oberer und unterer Gleitpartner mit einer konkaven inneren Artikulationsfläche (Konkavität) ausgebildet sind und die Geometrie dieser Konkavitäten durch eine zur Konvexität der Ober- und Unterseite des mittleren Gleitpartners korrespondierende Ausnehmung definiert ist, welche jeweils vollständig von einem Rand umschlossen wird, und
c) wenigstens ein Gleitpartner (11, 12, 13) einen zur gezielten Begrenzung der maximal möglichen Bewegung der Gleitpartner (11, 12, 13) geeigneten Rand (18) aufweist, der aufgrund seiner variierenden Höhe Konvexität (16) oder Konkavität (17) wellenförmig umgibt, und
d) ein anderer Gleitpartner (11, 12, 13) einen die korrespondierende Konkavität (17) oder Konvexität (16) umgebenden Rand (18) aufweist, der um die korrespondierende Konkavität (17) oder Konvexität (18) korrespondierend zum artikulierenden Gleitpartner (11, 12, 13) wellenförmig verläuft oder plan ist,
e) wobei die Höhenunterschiede eines wellenförmigen Randes (18) fließend und/oder in ein oder mehreren Stufen ineinander übergehen, und
f) es bei endgradiger Neigung und/oder Rotation der Gleitpartner (11, 12, 13) zueinander zu einer definierten Begrenzung der maximal möglichen Bewegung infolge Lückenschlusses der Ränder (18) kommt.

Beiden Prothesen ist gemeinsam, dass sie aus artikulierenden Gleitpartnern bestehen, von denen der jeweils obere Gleitpartner fest mit einem oberen Wirbelkörper und der jeweils untere Gleitpartner fest mit einem unteren Wirbelkörper verbunden ist, und wobei die Gleitpartner auf ihren zueinander gerichteten Innenseiten mit ineinandergreifenden Artikulationsflächen ausgebildet sind. Oberer und unterer Gleitpartner einer dreiteiligen Prothese sowie die beiden Gleitpartner einer zweiteiligen Prothese fungieren gleichzeitig als Abschlussplatten, welche Mittel aufweisen, die zur Verbindung mit einem oberen bzw. unteren Wirbelkörpern dienen.

Wegen der engen anatomischen Raumverhältnisse ist die zweiteilige Bandscheibenendoprothese in erster Linie für die Halswirbelsäule vorgesehen. Die zweiteilige Prothese kann aber auch für die Lendenwirbelsäule vorteilhaft sein wegen der modellimmanenten Stabilität bei Prothesenimplantationen in mehrere übereinander befindliche Bandscheiben. Die dreiteilige Bandscheibenendoprothese hat den Vorteil, dass das Transversalgleiten zweier benachbarter Wirbel nur minimal ist, mit dadurch hervorgerufener besonders vorteilhafter Adaptation an die Biomechanik des Bewegungssegments insbesondere der Lendenwirbelsäule. Mit der dreiteiligen Prothese kann zudem das inkonstante Rotationszentrum simuliert werden.

Im Zusammenhang mit der vorliegenden, Erfindung werden die drei Körperachsen durch die folgenden Begriffe bezeichnet: Ein Sagittalschnitt oder eine Ansicht in der sagittalen Ebene erlaubt eine Seitenansicht, da die zugrunde liegende Schnittebene senkrecht von vom nach hinten verläuft. Für die Angabe "vorn" wird auch "ventral" und für die Angabe "hinten" analog "dorsal" verwendet, da dies die Orientierung einer Prothese im Körper anzeigt. Ein "Frontalschnitt" oder die "frontale Ebene," ist ein senkrechter Querschnitt von einer Seite zur anderen Seite. Für die Angabe "seitlich" wird auch der Begriff "lateral" verwendet. Sowohl Sagittal- als auch Frontalschnitt sind Vertikalschnitte, da sie entlang einer vertikalen Ebene verlaufen, jedoch um 90 Grad versetzt zueinander orientiert sind. Eine Ansicht in "transversaler Ebene" oder ein "Transversalschnitt" erlaubt eine Aufsicht auf die Prothese, da es sich um einen Horizontalschnitt handelt.

In Verbindung mit der Beschreibung und Darstellung der vorliegenden Erfindung wird unter einer Artikulationsfläche der Bereich von Gleitpartnern verstanden, der aus den gekrümmten konvexen und konkaven Teilen der Oberflächen besteht, welche in Kontakt kommen und miteinander oder aufeinander gleiten bzw. artikulieren. Aus diesem Grund wird für Artikulationsfläche auch gleichbedeutend die Bezeichnung Gleitfläche verwendet.

Der Begriff korrespondierend bezeichnet im Zusammenhang mit artikulierenden Gleitflächen nicht ausschließlich kongruente konvexe und konkave Flächen, die miteinander artikulieren. Vielmehr werden damit auch miteinander artikulierende Gleitflächen bezeichnet, deren Oberflächen nicht vollständig kongruent sind. Derartige "Abweichungen" bzw. Toleranzen bezüglich der Gleitflächen artikulierender Gleitpartner können zum Einen durch die gewählten Materialien und Formen bedingt sein. Andererseits kann es aber auch beabsichtigt sein, dass Konvexität und damit artikulierende Konkavität nicht vollständig kongruent sind, um beispielsweise die zueinander gewünschten Bewegungsmöglichkeiten der Artikulationspartner gezielt vorgeben zu können.

Unter einem Rand soll im Sinne der vorliegenden Erfindung eine Fläche verstanden werden, welche sich zwischen Außenkante des jeweiligen Gleitpartners und Konvexität(en) bzw. Konkavität(en) befindet.

Bei einer zwei- und dreiteiligen erfindungsgemäßen Bandscheibenendoprothese sind die Konkavitäten von oberem und unterem Gleitpartner jeweils von einem Rand umschlossen, wohingegen sich die Konvexitäten des mittleren Gleitpartners einer dreiteiligen Prothese jeweils über die gesamte Ober- und Unterseite erstrecken, das heißt die Konvexitäten randfrei sind, oder die Konvexitäten jeweils von einem Rand umgeben sind. Die Ränder sind jeweils in ihrer gesamten Breite rundum gleich oder unterschiedlich hoch oder wellenförmig ausgestaltet, wobei die Höhenunterschiede eines wellenförmigen Randes fließend und/oder in ein oder mehreren Stufen ineinander übergehen. Weist bei einer dreiteiligen Bandscheibenendoprothese der mittlere Gleitpartner einen wellenförmigen Rand auf, so können die Ränder von oberem und unterem Gleitpartner ohne wellenförmige Ausgestaltung sein, da die Wellenform des Randes des mittleren Gleitpartners ausreicht, um die maximalen Bewegungsmaße der Gleitpartner nach ventral, dorsal und beidseits lateral festzulegen.

Wesentlich für die Gestaltung der Oberfläche der Ränder ist es, dass es bei einer endgradigen Neigung der Gleitpartner zueinander zu einem möglichst großflächigen Lückenschluss zwischen den Rändern der Gleitpartner kommt. Sofern die Ränder keine plane Oberfläche aufweisen, sind diese Ränder jedenfalls so gestaltet, das es bei einem Lückenschluss zu einem möglichst großflächigen Kontakt der Ränder kommt. In diesen Fällen kann ein Rand eine Wellenform aufweisen, die eine Konvexität oder Konkavität bis hin zur Außenkante vom Rand umgibt, oder auch eine Wellenform, die zur vorherigen um beispielsweise 90 Grad (wobei dies nicht ausschließlich und abschließend ist) gedreht ist und von innen nach außen geht. Unter der zuletzt genannten Wellenform, von innen nach außen, soll im Sinne der Erfindung auch eine kreis-, bogen- oder kurvenförmige Gestaltung des Randes verstanden werden.

Sowohl für eine zweiteilige als auch für eine dreiteilige erfindungsgemäße Bandscheibenendoprothese ergeben sich, bei erfindungsgemäßer Gestaltung der Randbereiche der Gleitpartner, Ansichten für wenigstens einen Gleitpartner, bei dem eine wellenförmige Gestaltung des Randes erkennbar ist, da die jeweils unterschiedlich hohen Randbereiche fließend und/oder in ein oder mehreren Stufen ineinander übergehen. Erfindungsgemäß ist für beide Prothesen bevorzugt vorgesehen, dass durch die wellenförmige Ausgestaltung von wenigstens einem Rand eines Gleitpartner die maximal mögliche Bewegung der Gleitpartner zueinander in dorsoventraler Richtung immer größer ist, als in laterolateraler Richtung.

Wesentlicher Vorteil von beiden erfindungsgemäßen Bandscheibenendoprothesen gegenüber den aus-dem Stand der Technik bereits bekannten Prothesen ist, dass bei großflächiger zentraler Druckübertragung infolge der sphärischen Gleitfläche(n), die maximal mögliche Neigung der Gleitpartner zueinander in laterolateraler und dorsoventraler Richtung durch die erfindungsgemäße Gestaltung der unterschiedlich hohen Randbereiche, deren Breite ringsherum gleich oder unterschiedlich ist, definiert eingestellt wird. Die unterschiedlich hohen Randbereiche gehen fließend und/oder in ein oder mehreren Stufen ineinander über und es ist allein durch den sich daraus ergebenden wellenförmigen Rand von wenigstens einem Gleitpartner möglich, die maximale laterolaterale Bewegungsmöglichkeit nur in einem geringeren Umfang zuzulassen, als die maximal mögliche Neigung der Gleitpartner zueinander in dorsoventraler Richtung. Dies entspricht der natürlichen Situation eines intakten Bewegungssegmentes einer gesunden Lendenwirbelsäule, bei der das Vor-/Rückneigen immer in einem deutlich stärkeren Ausmaß möglich ist, als das Seitneigen. Somit wird durch die erfindungsgemäßen Prothesen in einem Bewegungssegment eine Situation hinsichtlich der maximal möglichen Neigungswinkel in den verschiedenen Richtungen geschaffen, welche den physiologischen Bewegungsmöglichkeiten des Vor-, Rück- und Seitneigens sehr nahe kommt.

Es ist auch denkbar, dass durch die Gestaltung der Randbereiche einer erfindungsgemäßen Bandscheibenendoprothese die Neigungswinkel gezielt an die Notwendigkeiten angepasst werden, die bei einem Patienten zu beachten sind, dem die Prothese implantiert werden soll. So könnte durch die erfindungsgemäße Gestaltung der Randbereiche im Extremfall auch ein lateraler Neigungswinkel von 0 Grad eingestellt werden. Da die erfindungsgemäße Prothese jedoch eine möglichst physiologische Beweglichkeit haben soll und eine nicht mehr mögliche Seitneigung nicht physiologisch ist, stellt die 0°-Beweglichkeit also nur eine extreme Ausnahme einer Randgestaltung dar.

Bei einer dreiteiligen erfindungsgemäßen Prothese ist es auch vorgesehen, dass die maximal möglichen Neigungswinkel von oberem und unterem Gleitpartner allein durch die wellenförmigen Ränder des mittleren Gleitpartners eingestellt werden. Daraus ergibt sich die Möglichkeit zur Anpassung an Veränderungen der Wirbelsäule, lediglich den mittleren Gleitpartner gegen einen Gleitpartner mit anders ausgestalteten Rändern auszutauschen. Die mit den Wirbelkörpern verbundenen Gleitpartner müssen nicht ausgetauscht werden, sondern können im Patienten verbleiben, wodurch eine Schädigung der Wirbelkörper durch die Entfernung ausgeschlossen wird.

Durch die jeweilige erfindungsgemäße Begrenzung der Bewegungsmöglichkeit durch die Ränder wird vorteilhafterweise eine besondere Schonung der Wirbelbogengelenke erreicht, da diese eine überproportionale Beweglichkeit im Zwischenwirbelraum, wie dies bei Prothesen mit sphärischen Gleitflächen und stets gleich hohem Rand der Gleitpartner der Fall ist, nicht mehr ausgleichend begrenzen müssen. Demzufolge kann eine Über- bzw. Fehlbeanspruchung der Wirbelbogengelenke weitgehend vermieden werden.

Bezüglich der Rotation der Gleitpartner zueinander um eine gedachte vertikale Achse, kann je nach Gestaltung der Randbereiche bzw. der Ausgestaltung der wellenförmigen Bereiche und der Lage der jeweiligen Maxima dieser "Wellen" theoretisch eine freie Rotation der Gteitpartner zueinander möglich sein, wenn beispielsweise nur ein Gleitpartner einen wellenförmigen Rand aufweist. Sind jedoch die Randbereiche beider Gleitpartner einer Gleitfläche wellenförmig gestaltet, so ist es möglich, dass eine bestimmte Bewegung zugelassen wird und dann eine sanfte Begrenzung der Rotation stattfindet, da die Erhebungen und Vertiefungen der Ränder gegeneinander verdreht werden und die Prothese gegen den auf den Gleitpartnern lastenden Druck "aufgedreht" wird, vergleichbar mit der Führung eines Schraubverschlusses, bei dem auch Steigungen gegeneinander verdreht werden. Die Begrenzung der Rotation führt zu einer verbesserten physiologischen Situation im Bewegungssegment, insbesondere bei den Wirbelbogengelenken, denn *in vivo* ist eine Axialrotation in nur sehr geringem Ausmaß möglich.

Bei einer erfindungsgemäßen Bandscheibenendoprothese ist der maximal mögliche Neigungswinkel (gleichzeitig Öffnungswinkel) der Gleitpartner zueinander abhängig von der Höhe der sphärischen Konvexitäten in Bezug auf den sie umgebenden Rand sowie der jeweiligen Höhe, Neigung und wellenförmigen Ausgestaltung des die Konvexitäten und Konkavitäten umgebenden Randes.

Bezüglich des Materials ist bei einer erfindungsgemäßen Bandscheibenendoprothese vorgesehen, dass die Gleitpartner einstückig ausgebildet sind oder wenigstens ein Gleitpartner aus zwei fest oder fest, aber reversibel miteinander verbundenen Teilen besteht, wobei entweder die Konvexität(en) und/oder Konkavität(en) den Teil ausmachen, der fest oder fest, aber reversibel mit dem jeweiligen Gleitpartner verbunden ist, oder Konvexität(en) und/oder Konkavität(en) an der Basis geeignete Mittel für eine feste oder feste, aber reversible Verbindung aufweisen, wobei die miteinander verbundenen Teile aus gleichen oder verschiedenen Materialien bestehen oder die Oberflächen gleich oder verschieden beschichtet sind. Als geeignete Mittel für eine Verbindung sind erfindungsgemäß Anpassungen der Form der miteinander verbundenen Teile oder der Konvexität oder Konkavität entgegengesetzten Seite, wie beispielsweise flächige Verbreiterungen, die Teil des Randes oder der gesamte Rand sind, oder Ausnehmungen, vorgesehen. Als Teile, die abhängig von der jeweiligen Ausführungsform miteinander verbunden sein können, sind der jeweilige Gleitpartner und/oder Konvexität und/oder Konkavität sowie der Rand vorgesehen. Bei einem mittleren Gleitpartner ist zudem vorgesehen, dass dieser erst aus der Verbindung der jeweiligen Teile entsteht.

Sofern eine erfindungsgemäße Bandscheibenendoprothese aus fest oder fest, aber reversibel miteinander verbundenen Teilen besteht, ist für die Verbindung zwischen Gleitpartner und Konvexität(en) oder Konkavität(en) vorzugsweise eine Nut/Feder-Verbindung, eine Führungsschiene und korrespondierende Ausnehmung, ein Schnappmechanismus, Kleben oder Verschrauben vorgesehen.

Bei einer dreiteiligen erfindungsgemäßen Bandscheibenendoprothese ist erfindungsgemäß auch vorgesehen, dass oberer und unterer Gleitpartner aus dem gleichen Material bestehen oder gleich beschichtet sind und der mittlere Gleitkörper aus einem anderen Material gefertigt oder anders beschichtet ist.

Die Gleitpartner werden aus in der Implantattechnik bewährten Materialien gefertigt; beispielsweise bestehen der obere und untere Gleitpartner aus nichtrostendem Metall und der mittlere Gleitpartner aus medizinischem Polyäthylen. Andere Materialkombinationen sind denkbar. Die Verwendung anderer alloplastischer Materialien, die auch bioaktiv sein können, ist ebenfalls denkbar. Die Gleitpartner sind an den zueinander gerichteten Berührungsflächen hochglanzpoliert, um den Abrieb zu minimieren (low-friction-Prinzip). Im übrigen ist auch eine Beschichtung der einzelnen Gleitpartner mit geeigneten Materialen vorgesehen. Bevorzugt sind folgende Materialien vorgesehen: Titan, Titanlegierungen oder Titancarbid, Legierungen aus Kobalt und Chrom oder anderen geeigneten Metallen, Tantal oder geeignete Tantalverbindungen, geeignete keramische Materialien sowie geeignete Kunststoffe oder Verbundwerkstoffe.

In einer bevorzugten Ausführungsform der erfindungsgemäßen dreiteiligen Bandscheibenendoprothese weisen die kugelkappenförmigen Konvexitäten auf Ober- und Unterseite des mittleren Gleitpartners sowie die korrespondierenden Konkavitäten in oberem und unterem Gleitpartner identische oder verschiedene Radien auf. Dadurch werden die Möglichkeiten zur Adaptation der Bewegungsradien einer erfindungsgemäßen Bandscheibenendoprothese an die physiologischen Bewegungsradien erweitert.

Sowohl bei identischen als auch bei verschiedenen Krümmungsradien auf Ober- und Unterseite eines mittleren Gleitpartners ist die maximale Höhe der Konvexitäten auf Ober- und Unterseite des mittleren Gleitpartners gleich oder unterschiedlich. Abhängig von der Ausführungsform ist die Höhe des Randes eines mittleren Gleitpartners um einen gleichen Betrag wie die Höhe der Konvexität(en) verringert, oder die Höhe des Randes bleibt gleich oder ist different zur Höhenänderung der Konvexität(en), wobei die maximale Höhe der Konvexitäten auf Ober- und Unterseite dadurch gleich oder unterschiedlich ist.

Durch diese erfindungsgemäße Maßnahme wird es möglich, dass die Höhe der gesamten Prothese deutlich verringert wird. Eine derart flachere Prothese ist insbesondere für die Implantation im Halswirbelsäulenbereich vorgesehen. Es ist aber auch denkbar, dass eine derart flache Prothese im Lendenwirbelbereich eingesetzt wird, falls die dort vorhandenen Bandscheibenräume nur eine geringe Höhe aufweisen. Insgesamt wird durch diese erfindungsgemäße Maßnahme auch die Möglichkeit eröffnet, die Prothese durch die Wahl eines entsprechend hohen mittleren Gleitpartners an die Höhe eines Bandscheibenraumes anzupassen.

Durch die erfindungsgemäße Bandscheibenendoprothese ist ein Einstellen der maximalen Öffnungswinkel bei einseitigem Lückenschluss der Gleitpartner bei Extension oder Flexion zwischen 6 und 10 Grad und bei einseitigem lateralen Lückenschluss zwischen 0 und 6 Grad vorgesehen, wobei die 0 Grad Seitneigung eher eine Extremgestaltung darstellt und wenigstens 3 Grad Seitneigung durch die erfindungsgemäße Prothese ermöglicht werden sollen. Adaptiert an die Lenden- bzw. Halswirbelsäule können die konkreten maximalen Bewegungsmaße konstruktiv angepasst werden, ohne für jede einzelne Bandscheibe eine "eigene Prothese" vorsehen zu wollen. Die Öffnungswinkel entsprechen der natürlichen Segmentbeweglichkeit und werden durch eine geeignete Wahl der Konvexitäten und Konkavitäten in Zusammenhang mit der Ausgestaltung der diese umgebenden Wellen der Ränder erreicht (s.o.). Zum Ausgleich von Toleranzen im Bewegungssegment werden zusätzliche 3 Grad in jede Bewegungsrichtung einbezogen.

Sowohl bei einer erfindungsgemäß funktionell zwei- als auch bei einer funktionell dreiteiligen Bandscheibenendoprothese ist durch die erfindungsgemäße Ausgestaltung der Ränder eine begrenzte Rotationsbewegung der Gleitpartner zueinander um eine gedachte zentrale Vertikalachse von bis zu 3 Grad für die Lendenwirbelsäule und von bis zu 6 Grad für die Halswirbelsäule nach jeder Seite vorgesehen. Zum Ausgleich von Toleranzen im Bewegungssegment werden zusätzliche 2 Grad nach jeder Seite einbezogen.

Ferner ist bei einer zwei- und dreiteiligen erfindungsgemäßen Bandscheibenendoprothese vorgesehen, dass Konvexität und zugehörige korrespondierende Konkavität um bis zu 4 mm vom mittigen Frontalschnitt nach dorsal versetzt sind. Ein nach dorsal versetztes Rotationszentrum entspricht vor allem der physiologischen Situation im Übergang zwischen der Lendenwirbelsäule und dem Kreuzbein und andererseits werden so parallel die der physiologischen Situation entsprechenden Unterschiede zwischen den möglichen Neigungswinkeln bei der Extension und Flexion erreicht.

Des weiteren ist erfindungsgemäß vorgesehen, dass die Ränder der Gleitpartner nach außen rechtwinklig, anderweitig winklig, gekrümmt oder kombiniert gerade, gekrümmt und/oder winklig abgeschlossen sind. Insbesondere bei einer dreiteiligen Prothese mit Rand des mittleren Gleitkörpers ist in einer derartigen Ausführungsform eine Prothese denkbar, bei welcher Ober- und Unterseite des mittleren Gleitpartners im äußeren Randbereich einfach rechtwinklig oder gekrümmt miteinander abschneiden und die Randbreite nicht wesentlich anders als bei oberem und unterem Gleitpartner ausgestaltet ist. Somit wird der mittlere Gleitpartner auch bei endgradiger Neigung noch zwischen dem oberen und unteren Gleitpartner verbleiben und dadurch wird eine sehr kompakte und platzsparende Bauweise einer erfindungsgemäßen Bandscheibenendoprothese ermöglicht.

Bei dieser "kompakten" Ausführungsform einer erfindungsgemäßen dreiteiligen Bandscheibenendoprothese wird ein Herausgleiten des mittleren Gleitpartners einerseits durch die Höhen der Konvexität auf Ober- und Unterseite und der korrespondierenden Konkavitäten ab dem Rand rund-um die Artikulationsflächen und andererseits durch den Lückenschluss der Ränder der Gleitpartner bei endgradiger Neigung verhindert. Die Konvexitäten und die Wellenform im Randbereich sind derart gestaltet, dass die Konvexitäten tief genug in die artikulierenden Konkavitäten "eingreifen", insbesondere beim Rückneigen. Ein ausreichendes Aufspreizen der gesamten Prothese postoperativ, welches für das Herausgleiten des mittleren Gleitpartners Voraussetzung wäre, ist somit nicht möglich.

Weiterhin ist es erfindungsgemäß vorgesehen, dass bei einer dreiteiligen Prothese mit Rand des mittleren Gleitpartners zur zusätzlichen Sicherung gegen ein Herausgleiten, Abgleiten bzw. Herausrutschen (Luxation) bei Lückenschluss aller drei Gleitpartner, ein Anschlag Teil des Randes des mittleren Gleitpartners ist, der außerhalb des oberen und/oder unteren Gleitpartners angeordnet ist, wobei der Anschlag wenigstens auf Ober- oder Unterseite höher ist als der Rand des mittleren Gleitpartners ist.

Dieser Anschlag zur zusätzlichen Sicherung gegen ein Herausgleiten, Abgleiten bzw. Herausrutschen (Luxation) kann erfindungsgemäß auch derart ausgestaltet sein, dass der Anschlag Teil des Randes des mittleren Gleitpartners ist, wobei dieser auf Ober- und/oder Unterseite höher als der Rand des mittleren Gleitpartners ist und innerhalb einer Nut des Randbereichs vom oberen und/oder unteren Gleitpartner mit dem notwendigen Spiel für die maximale Gleitbewegung der Gleitpartner geführt wird.

Unter einem Anschlag soll im Sinne der vorliegenden Erfindung eine nach außen gerichtete Fortführung des Randes eines mittleren Gleitpartners verstanden werden, welche aufgrund der jeweiligen Ausgestaltung geeignet ist, ein Herausgleiten des mittleren Gleitpartners aus den Konkavitäten des oberen und unteren Gleitpartners zu verhindern. Ein Anschlag muss den mittleren Gleitpartner dazu nicht vollständig umschließen, da dies zu Einschränkungen der maximalen Beweglichkeit aller Gleitpartner führen kann, sondern gegebenenfalls in definierten Abständen oder gegenüber von Positionen des Randes angeordnet sein, welche für ein Herausgleiten des mittleren Gleitpartners in Frage kommen. Sofern der Anschlag auf Ober- und Unterseite höher als der Rand des mittleren Gleitpartners ist, kann er beispielsweise wie eine Heftzwecke ausgestaltet sein, die mit der Nadelspitze von außen in den Rand des mittleren Gleitpartners gesteckt wurde, so dass der Kopf der Heftzwecke oben und unten über den Rand des mittleren Gleitpartners übersteht und bei einer endgradigen Neigung zur Position der Heftzwecke das Herausgleiten des mittleren Gleitpartners verhindert, indem er an dem oberen und unteren Gleitpartner "anschlägt".

Ist ein Anschlag zur Sicherung gegen ein Herausgleiten Teil des Randes eines Gleitpartner, so ist die Höhe der Konvexität unter Beachtung der Anatomie und Materialeigenschaften lediglich abhängig von den gewünschten maximalen Neigungswinkeln, auf welche diese auch Einfluss hat (s.o.).

Ein Anschlag zur Sicherung des mittleren Gleitpartners bei einer dreiteiligen Prothese ist vorteilhafterweise derart gestaltet, dass er bei einer endgradigen Neigung der Gleitpartner ebenfalls an dem Lückenschluss des Randes beteiligt ist. Dadurch kommt dem Anschlag nicht nur eine Sicherungsfunktion zu, sondern er dient zusätzlich der Vergrößerung der mit Druck belasteten Flächen im Falle der endgradigen Neigung der Gleitpartner, deren Vorteile bereits beschrieben wurden. Die Möglichkeit einer derartigen Gestaltung hängt aber entscheidend von der Außenform des oberen und unteren Gleitpartners und der jeweiligen Randbreite von Konvexität und Konkavität ab.

In einer weiteren Ausführungsform einer dreiteiligen Bandscheibenendoprothese ist vorgesehen, dass die Höhe des Randes vom mittleren Gleitpartner ab dem Übergangsbereich zwischen der Konvexität und dem Rand bis zum äußeren Randbereich teilweise oder insgesamt kontinuierlich größer wird, ohne dass sich die Größe der Öffnungswinkel infolge Anpassung der Randhöhe des oberen und unteren Gleitpartners ändert. Diese "Schwalbenschwanzform" vom Rand des mittleren Gleitpartners erhöht dessen Sicherheit entgegen einer Dislokation.

Erfindungsgemäß ist bei dreiteiligen Prothesen auch eine Form von oberem und/oder unterem Gleitpartner vorgesehen, bei dem äußere Randbereiche vollständig oder partiell hakenförmig, rechtwinklig, anderweitig winklig, gekrümmt oder in Kombinationen davon in Richtung des anderen äußeren Gleitpartners ausgestaltet sind. Der Rand des mittleren Gleitpartners ist dort bei dieser Ausführungsform schmaler, so dass der mittlere Gleitpartner partiell oder vollständig von den Vorrichtungen eines oder beider äußerer Gleitpartner eingefasst wird, um ein Herausgleiten des mittleren Gleitkörpers zu verhindern. Vorteilhafterweise ist der Rand des mittleren Gleitpartners derart an die Randform eines äußeren Gleitpartners angepasst, dass bei einem endgradigen Lückenschluss eine möglichst große Fläche der artikulierenden Gleitpartner in Kontakt kommt.

Ferner ist bei der erfindungsgemäßen Bandscheibenendoprothese vorgesehen, dass sich der Außenumfang des oberen und unteren Gleitpartners in transversaler Ansicht von dorsal nach ventral (Lendenwirbelsäule) oder von ventral nach dorsal (Halswirbelsäule) verjüngen kann. Diese Verjüngung des Außenumfangs des oberen und unteren Gleitpartners kann lateral jeweils als identische Krümmung ausgebildet sein und ist bevorzugt Teilausschnitt eines Kreises. Fläche und Form des Außenumfanges des oberen und unteren Gleitpartners können je nach Bedarf gleich oder ungleich sein und so an die jeweilige Größe des Wirbelkörpers, mit dem sie verbunden werden, angepasst werden.

Die sich verjüngende Form von oberem und unterem Gleitpartner entspricht im wesentlichen der für die Prothesenplatten nutzbaren Fläche eines Wirbelkörpers in der Transversalansicht und führt so zu einer optimalen Nutzung der zur Verfügung stehenden Fläche eines Wirbelkörpers zur Verankerung von oberem und unterem Gleitpartner mit dem Ziel einer großflächigen Lastübertragung.

Ferner sind bei einer erfindungsgemäßen Bandscheibenendoprothese Gleitpartneranpassungen vorgesehen, wobei oberer und/oder unterer Gleitpartner im Frontalschnitt und/oder Sagittalschnitt derart ausgebildet sind, dass die Außen- und Innenseiten von oberem und/oder unterem Gleitpartner parallel oder eben nicht parallel zueinander verlaufen. Durch diese erfindungsgemäße Maßnahme kann eine erfindungsgemäße Bandscheibenendoprothese an Wirbelkörperendplatten angepasst werden, welche in Frontalansicht nicht parallel zueinander stehen bzw. in der Sagittalansicht eine optimale Lordose und Gleitflächenstellung zueinander ausbilden sollen.

Weiterhin ist vorgesehen, dass bei einer zwei- und dreiteiligen erfindungsgemäßen Ausführungsform die Konvexität (zweiteilige Prothese) bzw. der mittlere Gleitpartner (dreiteilige Prothese) bezüglich einer gedachten Horizontalen parallel oder nicht parallel ist. Bei einer nicht parallelen Ausführungsform stehen Ober- und Unterseite in einem Winkel in Bezug zur gedachten Horizontalen zueinander, wobei bei einem mittleren Gleitpartner der Winkel oben und unten gleich groß oder unterschiedlich sein kann. Die Konvexität(en) und korrespondierende(n) Konkavität(en) sind bei der zwei- und dreiteiligen Prothese in ihrer Oberflächengestaltung symmetrisch oder asymmetrisch. Durch die gewinkelte Konvexität bzw. den gewinkelten mittleren Gleitpartner sind ebenfalls Anpassungen an Asymmetrien des Wirbelkörperzwischenraumes möglich, in welchen die Prothese implantiert wird.

Zur sicheren Implantatverankerung im Zwischenwirbelraum dient eine randständige und/oder flächenhafte Verzahnung der Außenseiten von oberem und unterem Gleitpartner zur Verbindung mit einem oberen bzw. unteren Wirbelkörper. Die Außenseiten selbst sind plan oder konvex geformt und es ist möglich, die Verzahnung oder die gesamte Außenseite, auch ohne Verzahnung, bioaktiv oder stumpf zu beschichten. Um das Risiko einer Fraktur des Wirbelkörpers zu minimieren, ist eine Verankerung mit drei ventral angeordneten und zwei dorsal angeordneten Verankerungszähen bevorteilt. Alternativ sind durchgehende laterale Zahnreihen bevorteilt, zur besseren Führung des oberen und unteren Gleitpartners beim Einsetzen zwischen den Wirbelkörpern, da die Arbeitszange des Operateurs in die mittlere Lücke zwischen den Zahnreihen greifen kann oder auf Höhe der Zähne in Führungslöcher des oberen und unteren Gleitpartners.

Zur Vereinfachung der Im- oder Explantation der Bandscheibenendoprothese weisen oberer und/oder unterer Gleitpartner in einer weiteren Ausführungsform Vorsehungen für Instrumente auf. Diese Vorsehungen bestehen vorzugsweise aus Löchern oder Ausformungen, in die das jeweils benötigte Instrument des Operateurs eingreifen kann und so ein sicherer Halt des jeweiligen Gleitpartners ermöglicht wird.

Bei einer erfindungsgemäßen Bandscheibenendoprothese sind des weiteren als absolute Maße eine maximale Breite (Frontalansicht) von 14 bis 48 mm, eine maximale Tiefe (Sagittalschnitt) von 11 bis 35 mm und eine maximale Höhe von 4 bis 18 mm vorgesehenen. Diese Maße orientieren sich an den natürlichen Gegebenheiten der Lenden- und Halswirbelsäule und gewährleisten so, dass eine erfindungsgemäße Bandscheibenendoprothese der *in vivo* Situation möglichst nahe kommt.

Ferner sind bei einer erfindungsgemäßen Bandscheibenendoprothese ein oder mehrere röntgenkontrastgebende Markierungen vorgesehen, welche nicht röntgenkontrastgebende Teile der Prothese jeweils unterhalb ihrer Oberfläche enthalten. Dadurch ist es möglich, die Lage dieser Teile einer Bandscheibenendoprothese direkt nach der Implantation auf eine exakte Lage hin zu kontrollieren. Des weiteren ist es möglich, in definierten Zeitabständen durch Röntgen zu überprüfen, ob diese Teile der Prothese sich in ihrer Lage verändert haben bzw. immer noch exakt positioniert sind.

Weitere vorteilhafte Maßnahmen sind in den übrigen Unteransprüchen beschrieben; die Erfindung wird anhand von Ausführungsbeispielen und der nachfolgenden Figuren näher beschrieben; es zeigt:
- **Figur 1a - c**: Räumliche Frontalansichten einer funktionell dreiteiligen erfindungsgemäßen Bandscheibenendoprothese für die Lendenwirbelsäule:
a: Frontalansicht von dorsal
b: Frontalansicht von ventral
c: Mittiger Frontalschnitt
- **Figur 2 a, b**: Räumliche Sagittalansichten einer funktionell dreiteiligen erfindungsgemäßen Bandscheibenendoprothese:
a: Sagittalansicht
b: mittiger Sagittalschnitt
- **Figur 3a, b**: Räumliche Transversalansichten:
a: Innenseite von oberem oder unterem Gleitpartner
b: Innenseite von oberem oder unterem Gleitpartner mit aufliegendem mittleren Gleitpartner
- **Figur 4 a - c**: Schematische Darstellung verschiedener Formen des oberen und unteren Gleitpartners für die Lendenwirbelsäule;
- **Figur 5 a, b**: Schematische Darstellungen der Anordnung von Verankerungszähnchen auf den Außenseiten des oberen und unteren Gleitpartners für die Lendenwirbelsäule.
- **Figur 6a - e**: Schematische räumliche Darstellung verschiedener, wellenförmiger Gestaltungen eines mittleren Gleitpartners einer dreiteiligen erfindungsgemäßen Bandscheibenendoprothese.
- **Figur 7 a - g**: Schematische räumliche Darstellung einer dreiteiligen erfindungsgemäßen Bandscheibenendoprothese mit stufenförmiger Randgestaltung und Dislokationsschutz des mittleren Gleitpartners.

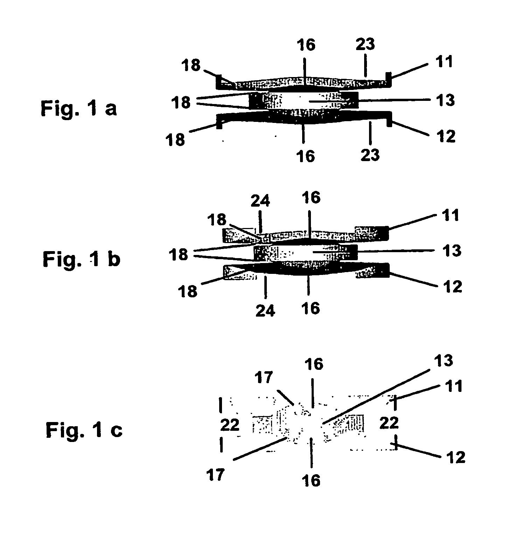

In den Figuren 1 a - c sind verschieden räumliche Frontalansichten einer Ausführungsform einer funktionell dreiteiligen erfindungsgemäßen Bandscheibenendoprothese für die Lendenwirbelsäule dargestellt, wobei in der dargestellten Ausführungsform der mittlere Gleitpartner 13 einen Rand 18 aufweist. Es sind erfindungsgemäß aber auch Ausführungsformen einer dreiteiligen Bandscheibenendoprothese vorgesehen, bei denen der mittlere Gleitpartner 13 ohne Rand 18 ausgebildet ist.

Die Figur 1 a zeigt eine Frontalansicht auf die dorsale Seite 23 und Figur 1b auf die ventrale Seite 24 einer erfindungsgemäßen Bandscheibenendoprothese. Bei einer erfindungsgemäßen Bandscheibenendoprothese, welche für die Implantation in den Lendenwirbelsäulenbereich vorgesehen ist, ist die ventrale Seite 24 (Figur 1 b) schmaler als die dorsale Seite 23 (Figur 1 a) aufgrund der sich verjüngenden Form von oberem und unterem Gleitpartner 11, 12 nach ventral. Sofern die Bandscheibenendoprothese für eine Implantation in den Halswirbelsäulenbereich vorgesehen ist, verjüngt sich der Außenumfang von oberem und unterem Gleitpartner 11, 12 nach dorsal.

Bei der in Figur 1 a und b dargestellten Ausführungsform ist die Höhe des Randes 18 des mittleren Gleitpartners 13 rund um die Konvexitäten 16 auf Ober- und Unterseite des mittleren Gleitpartners 13 gleich. Die Ränder 18 von oberem und unterem Gleitpartner 11, 12 sind lateral höher ausgebildet als dorsal und ventral. Die Übergänge zwischen den unterschiedlichen Höhen der Ränder 18 gehen fließend ineinander über, wodurch sich eine Wellenform der Ränder 18 ergibt. Diese Wellenform bedingt die gewünschte laterolateral geringere Neigungsmöglichkeit im Vergleich zur dorsoventralen Neigung der Gleitpartner 11, 12, 13 zueinander.

Figur 1c zeigt einen mittigen Frontalschnitt der in den Figuren 1 a und b dargestellten Ausführungsform einer erfindungsgemäßen Bandscheibenendoprothese. In diesem Schnitt ist deutlich zu erkennen, dass die lateralen Öffnungen 22 beidseits von Konvexität 16 des mittleren Gleitpartners 13 und Konkavität 17 von oberem und unterem Gleitpartner 11, 12 identisch sind.

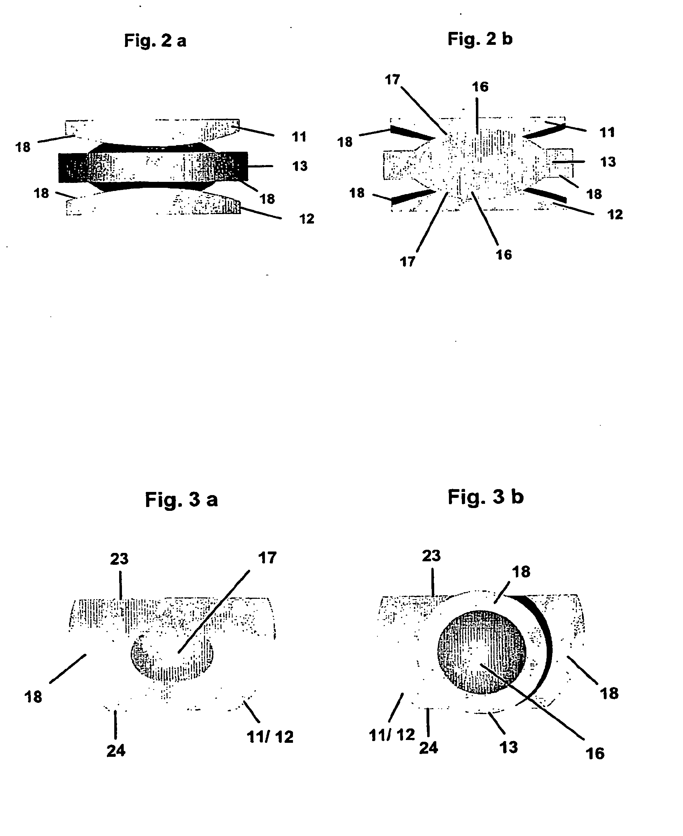

In den Figuren 2 a und b ist eine räumliche Sagittalansicht einer funktionell dreiteiligen erfindungsgemäßen Bandscheibenendoprothese zu sehen. In beiden Abbildungen ist jeweils der obere Gleitpartner 11, der untere Gleitpartner 12 sowie der dazwischen angeordnete mittlere Gleitpartner 13 dargestellt. In der dargestellten Ausführungsform weist der mittlere Gleitpartner 13 einen Rand 18 auf, hierbei handelt es sich um ein optionales Merkmal.

Figur 2 a zeigt eine sagittale Aufsicht auf die Lateralseite der Prothese. Der gleich hoch ausgestaltete Rand 18 des mittleren Gleitpartners 13 ist ebenso gut zu erkennen, wie der lateral jeweils höhere Rand 18 des oberen Gleitpartners 11 und des unteren Gleitpartners 12. Der Übergang von den lateral höheren Rändern 18 des oberen und unteren Gleitpartners nach dorsal und ventral ist fließend und ergibt in dieser Ansicht eine Bogenform. Sobald die Prothese jedoch Richtung dorsal oder ventral gedreht wird, ergibt sich für den oberen Rand 18 und den unteren Rand 18 eine Wellenform.

Figur 2b zeigt einen mittigen Sagittalschnitt der in Figur 2 a dargestellten erfindungsgemäßen Bandscheibenendoprothese mit oberem und unterem Gleitpartner 11, 12 und dazwischen angeordnetem mittleren Gleitpartner 13. Die Gleitpartner 11, 12, 13 artikulieren über die Konvexitäten 16 und die Konkavitäten 17. Da die Ränder 18 des oberen Gleitpartners 11 und unteren Gleitpartners 12 erfindungsgemäß dorsal und ventral nicht so hoch ausgestaltet sind wie lateral, ist im mittigen Sagittalschnitt der fließende Übergang der Ränder 18 als schwarze Fläche zu sehen. Da der Rand 18 des mittleren Gleitpartners 13 in der dargestellten Ausführungsform rundum in ganzer Breite gleich hoch ist, sind keine Übergänge im Rand zu sehen.

Figur 3 a zeigt eine Transversalansicht eines oberen oder unteren Gleitpartners einer erfindungsgemäß dreiteiligen Bandscheibenendoprothese 11, 12 oder den Gleitpartner mit Konkavität 17 einer erfindungsgemäß zweiteiligen Bandscheibenendoprothese. Die Außenform des Gleitpartners 11, 12 verjüngt sich bei der Lendenwirbelsäule von der dorsalen Seite 23 zur ventralen Seite 24, bei der Halswirbelsäule ist es umgekehrt. Es ist gut zu erkennen, dass es sich bei der Verjüngung der dargestellten Ausführungsform um Kreisabschnitte handelt. Die Unterschiede in der Höhe der Ränder 18 eines Gleitpartners 11, 12, welche dorsal und ventral nicht so hoch ausgestaltet sind wie lateral, sind in der Abbildung durch die Schattierung angedeutet.

In der Mitte, oder auch um bis zu 4 mm nach dorsal versetzt (nicht abgebildet), befindet sich die Konkavität 17. Diese ist korrespondierend zur kugelkappenförmigen Konvexität 16 (Figur 3 b) ausgestaltet. Aufgrund der unterschiedlich hohen Ränder 18, welche die Konkavität 17 vollständig umschließen, ergibt sich in der Aufsicht für die Konkavität keine kreisrunde Form, sondern eine dorsal und ventral abgeflacht runde Form.

In Figur 3b ist eine Transversalansicht eines oberen oder unteren Gleitpartners einer erfindungsgemäß dreiteiligen Bandscheibenendoprothese 11, 12 dargestellt, in dessen Konkavität ein mittlerer Gleitpartner 13 mit Rand 18 liegt. Auch in dieser Ansicht ist die sich verjüngende Außenform des oberen oder unteren Gleitpartners 11, 12 von der dorsalen Seite 23 zur ventralen Seite 24 (Lendenwirbelsäule) zu sehen. Durch die Schattierung der Ränder 18 sind die unterschiedlich hohen Randbereiche dorsoventral zu lateral angedeutet. Der Rand 18 des mittleren Gleitpartners 13 ist rund um die Konvexität 16 gleich hoch und aus diesem Grund unschattiert.

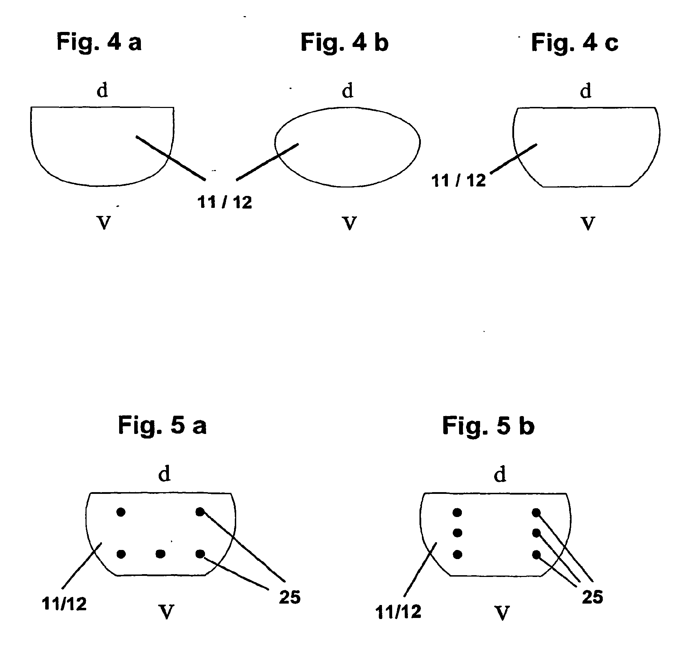

Die Figuren 4 a - c zeigen jeweils in einer Aufsicht auf oberen und unteren Gleitpartner 11, 12 schematisch alternative Gestaltungen der Form des Außenumfanges. Dabei ist mit den kleinen Buchstaben jeweils die Orientierung hinsichtlich der dorsoventralen Ausrichtung der Platten für die Lendenwirbelsäule angegeben (d = dorsal; v = ventral), die jedoch bei der Halswirbelsäule umgekehrt ist (v dann dorsal und d dann ventral).

In den Figuren 5 a und 5 b sind für die Lendenwirbelsäule alternative Anordnungen von Verankerungszähnchen 25 auf der Außenseite des oberen und unteren Gleitpartners 11, 12 dargestellt. Auch in dieser Zeichnung ist die Orientierung der Gleitpartner hinsichtlich der dorsoventralen Ausrichtung durch die kleinen Buchstaben kenntlich gemacht (d = dorsal; v = ventral). Dorsal ist in der Mitte jeweils kein Verankerungszähnchen 25 vorgesehen, da dies einerseits eine Schonung der Wirbelkörper bewirkt und andererseits die Implantation erleichtert. Für die Halswirbelsäule gilt wieder die entgegengesetzte Orientierung, ebenfalls ohne mittleres dorsales Verankerungszähnchen 25.

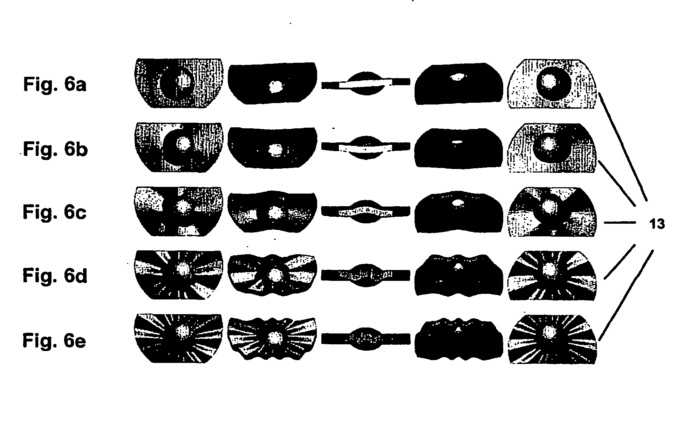

Die Figuren 6 a - e zeigen jeweils einen mittleren Gleitpartner 13 mit wellenförmiger Randgestaltung in verschiedenen räumlichen Ansichten. Der mittlere Gleitpartner 13 rotiert von links nach rechts jeweils um eine horizontale Drehachse um 180 Grad. Von Figur 6a, bei welcher der mittlere Gleitpartner nur eine Welle aufweist, nimmt die Anzahl der Wellen zur Figur 6e zu. Der Übergang der Wellen ineinander ist jeweils fließend.

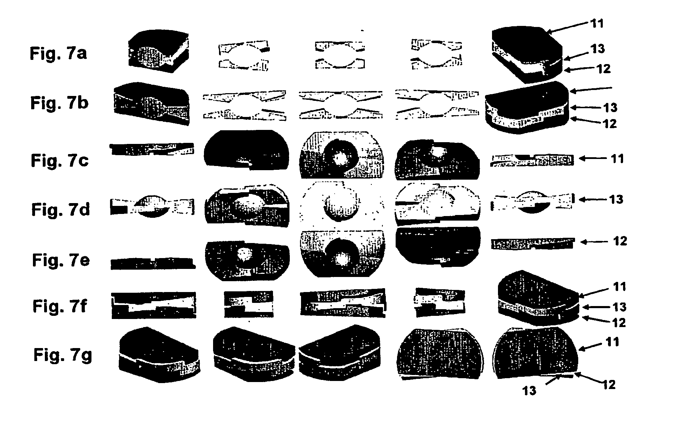

Die Figuren 7 a - g zeigen jeweils immer die gleiche Prothese mit stufenförmigen Wellen der Gleitpartner 11, 12, 13, wobei die Höhe des Randes des mittleren Gleitpartners 13 von der Konvexität zum Rand hin zunimmt. Durch diese Maßnahme soll eine Dislokation des mittleren Gleitpartners 13 aus der Prothese verhindert werden.

Figur 7a zeigt von links nach rechts die Prothese im mittigen Sagittalschnitt ohne Bewegung, (dabei ist links vorn und rechts ist hinten), bei Vomeigung, ohne Neigung, bei Rückneigung und in einer schrägen Draufsicht ohne Neigung (links ist hinten, rechts ist vom).

Figur 7b zeigt die Prothese in den gleichen Stellungen der Gleitpartner 11, 12, 13 im mittigen Frontalschnitt. Dabei entspricht dem Vor- und Rückneigen eine rechts- und linksseitige Neigung.

Die Figur 7c zeigt Vor- und Rückseite sowie die Innenseite des oberen Gleitpartners 11 in mehreren Ansichten.

Figur 7d zeigt Vor- und Rückseite sowie die Artikulationsflächen des mittleren Gleitpartners 13 in verschiedenen Ansichten und

Figur 7e zeigt den unteren Gleitpartner in Vor- und Rückansicht sowie die Innenseite in mehreren Ansichten.

Figur 7f zeigt die Prothese von vom, von der Seite, bei rechtsseitiger Neigung, bei Rückneigung und bei Neigung nach ventrolateral.

In Figur 7g ist die Prothese bei Rotationen dargestellt Ganz links ist eine Rotation zu sehen, bei der der mittlerer Gleitpartner 13 sich in Nullstellung befindet und der obere Gleitpartner 11 im Uhrzeigersinn rotiert und der untere Gleitpartner 12 entgegen dem Uhrzeigersinn. In der zweiten Abbildung von links ist die Rotation von oberem und unterem Gleitpartner 11, 12 umgekehrt. In der Mitte ist die Prothese ohne Rotation zu sehen. Die beiden Figurenteile rechts zeigen jeweils Draufsichten auf die links dargestellten Rotationen.

### Bezugszeichenliste

- 11: oberer Gleitpartner
- 12: unterer Gleitpartner
- 13: mittlerer Gleitpartner
- 16: Konvexität
- 17: Konkavität
- 18: Rand
- 22: Öffnung
- 23: dorsale Seite eines Gleitpartners (Lendenwirbelsäule)
- 24: ventrale Seite eines Gleitpartners (Lendenwirbelsäule)
- 25: Verankerungszähnchen

## Patentansprüche

1. Funktionell zweiteilige Bandscheibenendoprothese für den vollständigen Ersatz der Bandscheibe im Lenden- und Halswirbelsäulenbereich, bestehend aus einem oberen und einem unteren artikulierenden Gleitpartner, wobei der obere Gleitpartner eine obere Außenseite mit Mitteln für eine Verbindung mit einem oberen Wirbelkörper und der untere Gleitpartner eine untere Außenseite mit Mitteln für eine Verbindung mit einem unteren Wirbelkörper aufweist, und wobei die Gleitpartner auf zueinander gerichteten Innenseiten mit Artikulationsflächen ausgebildet sind, **dadurch gekennzeichnet, dass**
a) ein erster Gleitpartner (11, 12) derart ausgebildet ist, dass die zur Verbindung mit einem Wirbelkörper entgegengesetzte Seite eine Konvexität (16) aufweist und die Geometrie dieser Konvexität (16) einer Kugelkappe entspricht, wobei die Konvexität (16) vollständig von einem ersten Rand (18) umschlossen wird, und
b) ein zweiter Gleitpartner (11 oder 12) auf der zur Verbindung mit einem Wirbelkörper entgegengesetzten Seite eine Konkavität (17) aufweist und die Geometrie der Konkavität (17) durch eine zur kugelförmigen Konvexität (16) des ersten Gleitpartners (11 12) korrespondierende Ausnehmung definiert ist, wobei diese vollständig von einem zweiten Rand (18) umschlossen wird, und
c) entweder der erste oder der zweite Rand (18) wellenförmige zur gezielten Begrenzung der maximal möglichen Bewegung der Gleitpartner (11, 12) zueinander aufgrund seiner variierenden Höhe ausgestaltet ist, und
d) der jeweils andere Rand (18) des anderen Gleitpartners (11 oder 12) wellenförmig oder plan ausgestaltet ist,
e) wobei die Höhenunterschiede eines wellenförmigen Randes (18) fließend und/oder in ein oder mehreren Stufen ineinander übergehen, und
f) es bei endgradiger Neigung und/oder Rotation der Gleitpartner (11, 12) zueinander zu einer definierten Begrenzung der maximal möglichen Bewegung infolge Lückenschlusses der Ränder (18) kommt, und
g) durch die wellenförmige Ausgestaltung von wenigstens einem Rand (18) eines Gleitpartners (11, 12) die maximal mögliche Bewegung der Gleitpartner (11, 12) zueinander in dorsoventraler Richtung immer größer ist, als in laterolaterarler Richtung.

2. Funktionell dreiteilige Bandscheibenendoprothese für den vollständigen Ersatz der Bandscheibe im Lenden- und Halswirbelsäulenbereich, bestehend aus einem oberen, einem mittleren und einem unteren artikulierenden Gleitpartner, von denen der obere Gleitpartner eine obere Außenseite mit Mitteln zur Verbindung mit einem oberen Wirbelkörper und der untere Gleitpartner eine untere Außenseite mit Mitteln zur Verbindung mit einem unteren Wirbelkörper aufweist, und zwischen dem oberen und unteren Gleitpartner ein weiterer, mittlerer Gleitpartner mit einer Ober- und Unterseite angeordnet ist, wobei jeweils Artikulationsflächen zwischen der Innenseite des oberen Gleitpartners und der Oberseite des mittleren Gleitpartners sowie zwischen der Unterseite des mittleren Gleitpartners und der Innenseite des unteren Gleitpartner ausgebildet sind, wobei
a) der mittlere Gleitpartner (13) auf Ober- und Unterseite Konvexitäten (16) aufweist und die Geometrie dieser Konvexitäten (16) jeweils einer Kugelkappe entspricht, und
b) oberer und unterer Gleitpartner (11 , 12) auf der Innenseite mit einer Konkavität (17) ausgebildet sind und die Geometrie dieser Konkavitäten (17) durch korrespondierende Ausnehmungen zu den Konvexitäten (16) der Ober- und Unterseite des mittleren Gleitpartners (13) definiert ist, welche jeweils vollständig von einem Rand (18) umschlossen sind, und
c) entweder beim oberen oder beim unteren Gleitpartner (11, 12) der Rand (18) die Konkavität (17) wellenförmig umgibt und so zur gezielten Begrenzung der maximal möglichen Bewegung der Gleitpartner (11, 12, 13) zueinander aufgrund seiner variierenden Höhe ausgestaltet ist, und
d) der Rand (18) des jeweils anderen, oberen oder unteren, Gleitpartners (11, 12,) die Konkavität (17) wellenförmig oder plan umgibt
e) wobei die Höhenunterschiede eines wellenförmige Randes (18) fließend und/oder in ein oder mehreren Stufen ineinander übergehen, und
f) es bei endgradiger Neigung und/oder Rotation der Gleitpartner (11, 12, 13) zueinander zu einer definierten Begrenzung der maximal möglichen Bewegung infolge Lückenschlusses der Ränder (18) kommt, **dadurch gekennzeichnet, dass**
g) durch die wellenförmige Ausgestaltung von wenigstens einem Rand (18) des oberen oder unteren Gleitpartners (11, 12) die maximal mögliche Bewegung der Gleitpartner (11, 12 und 13) zueinander in dorsoventraler Richtung immer größer ist, als in laterolateraler Richtung.

3. Bandscheibeinendoprothese nach Anspruch 2, wobei beim mittleren Gleitpartner (13) die Konvexitäten (16) auf der Ober- und Unterseite von je einem Rand (18) umgeben sind, der jeweils in seiner gesamten Breite rundum gleich hoch oder unterschiedlich hoch oder wellenförmig ausgestaltet ist, wobei die Höhenunterschiede eines wellenförmigen Randes (18) fließend und/oder in ein oder mehreren Stufen ineinander übergehen.

4. Bandscheibenendoprothese nach wenigstens einem der vorhergehenden Ansprüche, wobei durch die wellenförmige Randgestaltung wenigstens eines Randes (18), nach ventral eine größere Neigung der Gleitpartner (11, 12 und 13) zueinander möglich ist als nach dorsal.

5. Bandscheibenendoprothese nach wenigstens einem der vorhergehenden Ansprüche, wobei der maximal mögliche Neigungswinkel (Öffnungswinkel) der Gleitpartner (11, 12, und 13) zueinander abhängig ist von
a) der Höhe der sphärischen Konvexität(en) (16), bei vorhandenem Rand in Bezug auf den sie umgebenden Rand (18), sowie
b) der jeweiligen Höhe, Neigung und wellenförmigen Ausgestaltung des die Konvexität(en) (16) und Konkavität(en) (17) umgebenden Randes (18).

6. Bandscheibenendoprothese nach wenigstens einem der vorhergehenden Ansprüche, wobei die Gleitpartner (11 , 12 und 13) einstückig ausgebildet sind.

7. Bandscheibenendoprothese nach wenigstens einem der Ansprüche 1 bis 5, wobei wenigstens ein Gleitpartner (11 , 12 und 13) aus wenigstens zwei fest oder fest, aber reversibel miteinander verbundenen Teilen besteht, wobei entweder die Konvexität(en) (16) und/oder Konkavitäten) (17) den Teil aus machen, der fest oder fest, aber reversibel mit dem jeweiligen Gleitpartner (11, 12 und 13) verbunden ist, oder Konvexität(en) (16) und/oder Konkavität(en) (17) an der Basis geeignete Mittel für eine feste oder feste, aber reversible Verbindung aufweisen.

8. Bandscheibenendoprothese nach wenigstens einem der vorhergehenden Ansprüche, wobei die Gleitpartner (11 , 12 und 13) und/oder miteinander verbundene Teile aus gleichen oder verschiedenen Materialien bestehen.

9. Bandscheibenendoprothese nach wenigstens einem der vorhergehenden Ansprüche, wobei die Oberflächen der Gleitpartner (11, 12 und 13) und/oder miteinander verbundenen Teile gleich oder verschieden beschichtet sind.

10. Bandscheibenendoprothese nach wenigstens einem der Ansprüche 7 bis 9, wobei eine feste oder feste, aber reversible Verbindung durch eine Nut/Feder-Verbindung, eine Führungsschiene und korrespondierende Ausnehmung, einen Schnappmechanismus, Kleben oder Verschrauben hergestellt ist.

11. Bandscheibenendoprothese nach wenigstens einem der Ansprüche 2 bis 10, wobei oberer und unterer Gleitpartner (11, 12) aus dem gleichen Material bestehen oder gleich beschichtet sind und der mittlere Gleitpartner (13) aus einem anderen Material gefertigt ist oder anders beschichtet ist.

12. Bandscheibenendoprothese nach Anspruch 2 oder wenigstens einem darauf rückbezogenen vorhergehenden Anspruch, wobei die kugelkappenformigen Konvexitäten (16) auf Ober- und Unterseite des mittleren Gleitpartners (13) sowie die korrespondierenden Konkavitäten (17) des oberen und unteren Gleitpartners (11, 12) identische oder unterschiedliche Radien aufweisen.

13. Bandscheibenendoprothese nach Anspruch 2 oder wenigstens einem darauf rückbezogenen vorhergehenden Anspruch, wobei die maximale Höhe der Konvexitäten (16) des mittleren Gleitpartners (13) auf Ober- und Unterseite gleich oder unterschiedlich ist.

14. Bandscheibenendoprothese nach wenigstens einem der vorhergehenden Ansprüche, wobei die maximale Höhe einer Konvexität (16) geringer ist als eine Halbkugel und/oder bei vorhandenem Rand die Höhe des Randes (18) verringert ist, wobei bei einer dreiteiligen Bandscheibenendoprothese die Höhe der Konvexitäten (16) auf Ober- und Unterseite gleich oder unterschiedlich ist.

15. Bandscheibenendoprothese nach wenigstens einem der vorhergehenden Ansprüche, wobei der maximale Öffnungswinkel bei einseitigem Lückenschluss der Gleitpartners (11, 12 und 13) aus der Extension oder Flexion zwischen 6 und 10 Grad und bei lateralen Lückenschluss zwischen 0 und 6 Grad beträgt, mit einer Toleranz von zusätzlich je 3 Grad in jede Richtung.

16. Bandscheibenendoprothese nach wenigstens einem der vorhergehenden Ansprüche, wobei die Ausgestaltung der Ränder (18) eine begrenzte Rotationsbewegung der Gleitpartner (11, 12 und 13) im Bezug auf eine gedachte zentrale Vertikalachse um bis zu 3° für die Lendenwirbelsäule und um 6° für die Halswirbelsäule nach jeder Seite ermöglicht, mit einer Toleranz von zusätzlich je 2 Grad nach jeder Seite.

17. Bandscheibenendoprothese nach wenigstens einem der vorhergehenden Ansprüche, wobei Konvexität(en) (16) und zugehörige korrespondierende Konkavität(en) (17) um bis zu 4 mm vom mittigen Frontalschnitt nach dorsal versetzt sind.

18. Bandscheibenendoprothese nach wenigstens einem der vorhergehenden Ansprüche, wobei die Ränder (18) der Gleitpartner (11 , 12, und 13) nach außen rechtwinklig, anderweitig winklig, gekrümmt oder kombiniert gerade, gekrümmt und/oder winklig abgeschlossen sind.

19. Bandscheibenendoprothese nach Anspruch 2 oder wenigstens einem darauf rückbezogenen vorhergehenden Anspruch, wobei zur zusätzlichen Sicherung eines mittleren Gleitpartners (13) mit Rand (18) gegen ein Herausgleiten aus der Prothese, beim Lückenschluss aller drei Gleitpartner (11 , 12, 13) ein Anschlag Teil des Randes (18) des mittleren Gleitpartners (13) ist, der außerhalb des oberen und/oder unteren Gleitpartners (11, 12) angeordnet ist, wobei der Anschlag wenigstens auf Ober- oder Unterseite höher ist als der Rand (18) des mittleren Gleitpartners (13).

20. Bandscheibenendoprothese nach Anspruch 2 oder wenigstens einem darauf rückbezogenen Anspruch, wobei zur zusätzlichen Sicherung eines mittleren Gleitpartners (13) mit Rand (18) gegen ein Herausgleiten aus der Prothese, beim Lückenschluss aller drei Gleitpartner (11, 12, 13), ein Anschlag Teil des Randes (18) des mittleren Gleitpartners (13) ist, der auf Ober- und/oder Unterseite höher als der Rand (18) des mittleren Gleitpartners (13) ist und innerhalb einer Nut des Randbereichs vom oberen und/oder unteren Gleitpartner (11, 12) mit dem notwendigen Spiel für die maximale Gleitbewegung der Gleitpartner (11, 12, 13) geführt wird.

21. Bandscheibenendoprothese nach Anspruch 2 oder wenigstens einem darauf rückbezogenen vorhergehenden Anspruch, wobei zur zusätzlichen Sicherung eines mittleren Gleitpartner (13) mit Rand (18) gegen ein Herausgleiten aus der Prothese, beim Lückenschluss aller drei Gleitpartner (11, 12, 13), der Rand (18) des mittleren Gleitpartner (13) ab dem Übergangsbereich der Konvexität nach peripher teilweise oder insgesamt kontinuierlich höher wird und der Rand des oberen und/oder unteren Gleitpartners (11, 12) im gleichen Maße flacher wird.

22. Bandscheibenendoprothese nach Anspruch 2 oder wenigstens einem darauf rückbezogenen vorhergehenden Anspruch, wobei zur zusätzlichen Sicherung eines mittleren Gleitpartners (13) gegen ein Herausgleiten aus der Prothese, beim Lückenschluss der drei Gleitpartner (11, 12, 13) die äußeren Kanten von oberem und/oder unterem Gleitpartner (11, 12) vollständig oder partiell hakenförmig, rechtwinklig, anderweitig winklig, gekrümmt oder in Kombinationen davon in Richtung des anderen äußeren Gleitpartners ausgestaltet sind.

23. Bandscheibenendoprothese nach wenigstens einem der vorhergehenden Ansprüche, wobei Fläche und Form des Außenumfanges des oberen und unteren Gleitpartners (11, 12) gleich oder ungleich sind und so an die jeweilige Größe des wirbelkörpers, mit dem sie verbunden, werden, angepasst sind.

24. Bandscheibenendoprothese nach wenigstens einem der vorhergehenden Ansprüche, wobei oberer und/oder unterer Gleitpartner (11, 12) im Frontal- und/oder Sagittalschnitt derart ausgebildet sind, dass die Außen- und Innenseite von oberem und/oder unterem Gleitpartner (11, 12) parallel oder nicht parallel zueinander verlaufen.

25. Bandscheibenendoprothese nach wenigstens einem der vorhergehenden Ansprüche, wobei die Konvexität einer zweiteiligen Prothese (11, 12) und Ober- und Unterseite des mittleren Gleitpartners (13) einer dreiteiligen Prothese in Bezug zu einer Horizontalen parallel oder nicht parallel sind, und dann zueinander in einem definierten Winkel stehen, wobei die Konvexität(en) symmetrisch oder asymmetrisch sind.

26. Bandscheibenendoprothese nach wenigstens einem der vorhergehenden Ansprüche, wobei der obere und untere Gleitpartner (11 , 12) auf der Außenseite plan oder konvex und bioaktiv beschichtet oder stumpf sind und zu deren Primärverankerung mit den Wirbelkörpern Reihen von Verankerungszähnchen (25) aufweisen, welche entweder von dorsal nach ventral seitlich gerade oder schräg angeordnet sind oder ventral und dorsal in lateraler Ausrichtung angeordnet sind, wobei sich in der dorsalen Reihe nur an den Seiten Verankerungszähnchen (25) befinden.

27. Bandscheibenendoprothese nach wenigstens einem der vorhergehenden Ansprüche, wobei der obere und/oder untere Gleitpartner (11, 12) Mittel zum Eingreifen eines Instrumentes zur Im- oder Explantation aufweisen.

28. Bandscheibenendoprothese nach wenigstens einem der vorhergehenden Ansprüche, wobei diese eine maximale Breite (Frontalansicht) von 14 bis 48 mm, eine maximale Tiefe (Sagittalschnitt) von 11 bis 35 mm und eine maximale Höhe von 4 bis 18 mm aufweist.

29. Bandscheibenendoprothese nach wenigstens einem der vorhergehenden Ansprüche 1 bis 28 für die Implantation in die Lendenwirbelsäule, wobei sich der Außenumfang des oberen und unteren Gleitpartners (11, 12) in transversaler Ansicht nach ventral verjüngt.

30. Bandscheibenendoprothese nach wenigstens einem der vorhergehenden Ansprüche 1 bis 28 für die Implantation in die Halswirbelsäule, wobei sich der Außenumfang des oberen und unteren Gleitpartners (11, 12) in transversaler Ansicht nach dorsal verjüngt.

31. Bandscheibenendoprothese nach Anspruch 29 oder 30, wobei die Verjüngung des Außenumfangs der Gleitpartner (11, 12) lateral jeweils als identische Krümmungen oder asymmetrisch ausgebildet sind.

32. Bandscheibenendoprothese nach einem der vorhergehenden Ansprüche, wobei die Prothese jeweils unterhalb der Oberfläche ein oder mehrere röntgenkontrastgebende Markierungen enthält.

## Claims

1. Functionally two-part intervertebral disc prosthesis for the total replacement of the intervertebral disc within the lumbar and cervical spine, comprising an upper and a lower articulating sliding partner, wherein the upper sliding partner has an upper surface with means for an assembly to an upper vertebral body and the lower sliding partner has a lower surface with means for a assembly to a lower vertebral body and wherein the sliding partners have with sliding areas on the inner sides that are directed towards each other, **characterized by**
a) a first sliding partner (11, 12) constructed in such a manner, that the opposite side of the side for the assembly with a vertebral body has a convexity (16), and the geometry of this convexity (16) corresponds to a cap of a sphere, wherein the convexity (16) is completely surrounded by a first edge(18), and
b) a second sliding partner (11 or 12), has a concavity (17) on the side opposite of that assembled to the vertebral body, and the geometry of the concavity (17) is defined by a recess corresponding with the spherical convexity (16) of the first sliding partner(11, 12) wherein the recess is completely surrounded by a second edge (18), and
c) either the first or the second, edge (18) is constructed in a wavelike manner for the targeted limitation of the maximal possible motion of the sliding partners (11, 12) towards each other, which is a result of its varying height, and
d) the respective other edge (18) of the other sliding partner (11 or 12) is constructed in a wavelike or plane manner,
e) with the differences of the heights of a wavelike edge (18) changing seamlessly and/or in one or several steps, and
f) that during terminal inclination and/or rotation of the sliding partners (11, 12) towards each other a definite limitation of the maximal possible motion arises as a result of a gap-closure of the edges (18), and
g) the maximal possible motion of the sliding partners (11, 12) towards each other is always greater in dorsoventral direction than in laterolateral direction due to the wavelike construction of at least one edge (18).

2. Functionally three-part intervertebral disc prosthesis for the total replacement of the intervertebral disc within the lumbar and cervical spine, comprising of an upper, a middle and a lower articulating sliding partner, wherein the upper sliding partner has an upper surface with means for a assembly to an upper vertebral body and the lower sliding partner has a lower surface with means for a assembly to a lower vertebral body and wherein between the upper and lower sliding partner a further, middle sliding partner with an upper and lower surface is located, wherein between the inner side of the upper sliding partner and the upper side of the middle sliding partner as well as between the lower side of the middle sliding partner and the inner side of the lower sliding partner, respectively, sliding areas are located, wherein
a) the middle sliding partner (13) has on the upper and lower side convexities (16), and the geometry of these convexities (16) each correlate with the cap of a sphere, and
b) upper and lower sliding partner (11, 12) are constructed with a concavity (17) on the inner side, and the geometry of these concavities (17) is defined by a corresponding recess to the convexities (16) of the upper and lower side of the middle sliding partner (13), each being totally surrounded by a edge (18), and
c) the edge (18) either of the upper or the lower sliding partner surrounds the concavity (17) in a wavelike manner edge and is thus constructed for a targeted limitation of the maximal possible motion of the sliding partners (11, 12, 13) towards each other, as a result or its varying height, and
d) the edge (18) of the respective other, upper or lower, sliding partner (11, 12) edge surrounds the concavity (17) in a wavelike or plane manner,
e) wherein the differences of the heights of a wavelike edge (18) change seamlessly and/or in one or several steps, and
f) that during terminal inclination and/or rotation of the sliding partners (11, 12, 13) towards each other a defined limitation of the maximal possible motion arises as a result of a gap-closure of the edges (18), **characterized by**
g) the maximal possible motion of the sliding partners (11, 12 and 13) towards each other is always greater in dorsoventral direction than in laterolateral direction due to the wavelike construction of at least one edge (18).

3. Intervertebral disc prosthesis according to claim 2, wherein the convexities (16) of the middle sliding partner (13) are each surrounded on the upper and lower side by an edge (18), which has the same height or is differently high or is wavelike shaped along its entire breadth, wherein the differences in heights of the wavelike edge (18) change seamlessly and/or in one or several steps.

4. Intervertebral disc prosthesis according to at least one of the preceding claims, wherein as the result of the wavelike edge shape of at least one edge (18) a greater inclination of the sliding partners (11, 12 and 13) towards each other is possible in ventral direction than in a dorsal direction.

5. Intervertebral disc prosthesis according to at least one of the preceding claims, wherein the maximal possible angle of inclination (aperture angle) of the sliding partners (11, 12 and 13) towards each other depends on
a) the height of the spherical convexity(ies)(16), in case that an edge is present with respect to the edge (18) surrounding it, as well as
b) the respective height, inclination and wavelike shape of the edge (18) surrounding the convexity(ies) (16) and concavity(ies) (17).

6. Intervertebral disc prosthesis according to at least one of the preceding claims, wherein the sliding partners (11, 12, 13) are constructed in one piece.

7. Intervertebral disc prosthesis according to at least one of claims 1 to 5, wherein at least one sliding partner (11, 12 and 13) consists of at least two permanent or permanently, but reversibly assembled parts, wherein either the convexity(ies) (16) and/or the concavity(ies) (17) are the parts that are permanent or permanently, but reversibly assembled to the respective sliding partners (11, 12 und 13), or the convexity(ies) (16) and/or concavity(ies) (17) have at their basis suitable means for a permanent or permanent, but reversible assembly.

8. Intervertebral disc prosthesis according to at least one of the preceding claims, wherein the sliding partners (11, 12 und 13) and/or with each other connected parts consist of the same or different materials.

9. Intervertebral disc prosthesis according to at least one of the preceding claims, wherein the surfaces of the sliding partners (11,12 and 13) and/or with each other connected parts are coated equally or differently.

10. Intervertebral disc prosthesis according to at least one of the claims 7 to 9, wherein a permanent or permanent, but reversible assembly is achieved by a tongue and groove assembly, a track and corresponding recess, a snap mechanism, gluing or screwing.

11. Intervertebral disc prosthesis according to one of the claims 2 to 10, wherein upper and lower sliding partner (11, 12) consist of the same material or are equally coated and the middle sliding partner (13) is made of a different material or is differently coated.

12. Intervertebral disc prosthesis according to claim 2 or at least one of the preceding claims referring to it, wherein the spherical cap-shaped convexities (16) on upper and lower side of the middle sliding partner (13) as well as the corresponding concavities (17) of the upper and lower sliding partner (11, 12) have identical or different radii.

13. Intervertebral disc prosthesis according to claim 2 or at least one of the preceding claims referring to it, wherein the maximal height of the convexities (16) of the middle sliding partner (13) is equal or different on the upper and lower side.

14. Intervertebral disc prosthesis according to at least one of the preceding claims, wherein the maximal height of a convexity (16) is less than a hemisphere and/or in case that an edge is present, the height of the edge (18) is reduced, wherein the height of the convexities (16) is equal or different on the upper and lower side in the case of a three-part intervertebral disc prosthesis.

15. Intervertebral disc prosthesis according to at least one of the preceding claims, wherein the maximal aperture angle at a one-sided gap-closure of the sliding partners (11, 12 and 13) during extension or flexion is between 6 und 10 degrees and at a lateral gap closure is between 0 und 6 degrees, with a tolerance of an additional 3 degrees in every direction.

16. Intervertebral disc prosthesis according to at least one of the preceding claims, wherein the shaping of the edges (18) allows for a limited rotational motion of the sliding partners (11, 12 and 13) with respect to a fictitious vertical axis of up to 3° for the lumbar spine and of 6° for the cervical spine to every side, with a tolerance of an additional 2 degrees to each side.

17. Intervertebral disc prosthesis according to at least one of the preceding claims, wherein the convexity(ies) (16) and the respective corresponding concavity(ies) (17) are dorsally displaced up to 4 mm away from a centric frontal section.

18. Intervertebral disc prosthesis according to at least one of the preceding claims, wherein the edges (18) of the sliding partners (11, 12 und 13) end outwardly perpendicular, otherwise angled, curved or have a combination of straight, curved, and /or angular.

19. Intervertebral disc prosthesis according to claim 2 or at least one of the preceding claims referring to it, wherein as an additional safeguard for a middle sliding partner (13) with edge (18) against a slip-out out of the prosthesis during a gap-closure of all three sliding partners (11, 12, 13) a stop is part of the edge (18) of the middle sliding partner (13), that is located outside the upper and/or lower sliding partner (11, 12), wherein the stop is at least on the upper or the lower side higher than the edge (18) of the middle sliding partner (13).

20. Intervertebral disc prosthesis according to claim 2 or at least one claim referring to it, wherein as an additional safeguard for the middle sliding partner (13) with edge (18) against a slip-out out of the prosthesis during a gap-closure of all three sliding partners (11, 12, 13) a stop is part of the edge (18) of the middle sliding partner (13), which is higher on the upper and/or the lower side than the edge (18) of the middle sliding partner (13) and is guided within a groove of the edge area of the upper and/or lower sliding partner (11, 12) with the necessary clearance for the maximal sliding motions of the sliding partners (11, 12, 13).

21. Intervertebral disc prosthesis according to claim 2 or at least one preceding claim referring to it, wherein as an additional safeguard for the middle sliding partner (13) with edge (18) against a slip-out out of the prosthesis during a gap-closure of all three sliding partners (11, 12, 13) the edge (18) of the middle sliding partner (13) partly or totally increases continuously in height from the transition area of the convexity to the periphery and the edge of the upper and/or lower sliding partner (11,12) levels off to the same degree.

22. Intervertebral disc prosthesis according to claim 2 or at least one preceding claim referring to it, wherein as an additional safeguard for the middle sliding partner (13) against a slip-out out of the prosthesis during a gap-closure of all three sliding partners (11, 12, 13), the most outward portion of the upper and/or lower sliding partner (11, 12) are completely or partly hook-shaped, perpendicular, otherwise angular, curved or a combination thereof in a direction of the other external sliding partner.

23. Intervertebral disc prosthesis according to at least one of the preceding claims, wherein surface and shape of the outer circumference of the upper and lower sliding partner (11, 12) are equal or unequal and are adapted by this to the corresponding size of the vertebral body with which they are to be assembled.

24. Intervertebral disc prosthesis according to at least one of the preceding claims, wherein upper and/or lower sliding partner (11, 12) are constructed in such a manner that in frontal and/or sagittal section the outside and inside of the upper and/or lower sliding partner (11, 12) run parallel or non parallel.

25. Intervertebral disc prosthesis according to at least one of the preceding claims, wherein the convexity of a two-part prosthesis (11, 12) and the upper and lower side of the middle sliding partner (13) of a three-part prosthesis are parallel or nonparallel with respect to a horizontal, and thus run in a defined angle relative to each other, with the convexity(ies) being symmetrical or asymmetrical.

26. Intervertebral disc prosthesis according to at least one of the preceding claims, wherein the upper and lower sliding partner (11, 12) are plane or convex and coated bio-actively or bluntly on the outer surface and have for their primary anchorage with the vertebral bodies teeth (25), that are either arranged from dorsal to ventral laterally straight or oblique or ventral and dorsal in straight direction, and in the dorsal row anchoring teeth (25) are arranged laterally only.

27. Intervertebral disc prosthesis according to at least one of the preceding claims, wherein the upper and/or lower sliding partner (11, 12) have means for an instrument to engage for implantation or explantation.

28. Intervertebral disc prosthesis according to at least one of the preceding claims, wherein it has a maximal breadth (frontal view) from 14 to 48 mm, a maximal depth (sagittal view) from 11 to 35 mm and a maximal height from 4 to 18 mm.

29. Intervertebral disc prosthesis according to at least one of the preceding claims 1 - 28 for the implantation into the lumbar spine, with the outer circumference of the upper and lower sliding partners (11, 12) tapering off ventrally in the transversal view.

30. Intervertebral disc prosthesis according to at least one of the preceding claims 1 - 28 for the implantation into the cervical spine, with the outer circumference of the upper and lower sliding partners (11, 12) tapering off dorsally in the transversal view.

31. Intervertebral disc prosthesis according to claims 29 or 30, wherein the diminution of the outer circumference of the sliding partners (11, 12), has laterally respective identical curvations or is asymmetric.

32. Intervertebral disc prosthesis according to at least one of the preceding claims, wherein the prosthesis has one or more X-ray contrast giving tags under their surface.

## Revendications

1. Endoprothèse de disque fonctionnelle en deux parties, pour le remplacement total du disque intervertébral dans la zone de la colonne lombaire et de la colonne cervicale, comprenant un palier de glissement d'articulation supérieur et un palier de glissement d'articulation inférieur, dans lequel le palier de glissement supérieur comprend un côté externe supérieur avec des moyens pour une liaison avec un corps vertébral supérieur, et le palier de glissement inférieur comprend un côté extérieur inférieur avec un moyen pour une liaison avec un corps vertébral inférieur, et dans laquelle les paliers de glissement sont conçus avec des surfaces d'articulation sur les côtés internes orientés l'un vers l'autre, **caractérisé en ce que**
a) un premier palier de glissement (11,12) est conçu de manière que le côté opposé au côté pour la liaison avec un corps vertébral comporte une convexité (16), et la géométrie de cette convexité (16) correspond à une calotte, la convexité (16) étant complètement entourée par une première bordure (18), et
b) un deuxième palier de glissement (11 ou 12) comprend une concavité (17) sur le côté opposé à celui pour la liaison avec un corps vertébral, la géométrie de la concavité (17) étant définie par un évidement correspondant à la convexité (16) sphérique du premier palier de glissement (11, 12), la concavité étant complètement entourée par une deuxième bordure (18), et
c) soit la première soit la deuxième bordure (18) est conçue sous forme ondulée pour une délimitation prédéfinie du mouvement maximal possible des paliers de glissement (11, 12) l'un vers l'autre, en fonction de sa hauteur variable, et
d) l'autre bordure respective (18) de l'autre palier de glissement (11 ou 12) est conçue sous forme ondulée ou sous forme plane,
e) les différences de hauteurs d'une bordure en forme ondulée (18) passent de manière fluide les unes dans les autres et/ou en une ou plusieurs marches, et
f) pour une inclinaison terminale et/ou une rotation des paliers de glissement (11, 12) l'un vers l'autre, une limitation définie du mouvement maximal possible a lieu suite à une fermeture d'espace des bordures (18), et
g) grâce à l'arrangement sous forme ondulée d'au moins une bordure (18) d'un palier de glissement (11, 12) le mouvement maximal possible des paliers de glissement (11, 12) l'un vers l'autre est toujours supérieur en direction dorsoventrale par rapport à en direction latéro-latérale.

2. Endoprothèse de disque fonctionnelle en trois parties pour le remplacement total du disque intervertébral dans la zone de la colonne lombaire et de la colonne cervicale, comprenant un palier de glissement d'articulation supérieur, un palier de glissement d'articulation intermédiaire, et un palier de glissement d'articulation inférieur, parmi lesquels le palier de glissement supérieur comprend un côté extérieur supérieur avec des moyens de fixation à un corps vertébral supérieur, et le palier de glissement inférieur comprend un côté extérieur inférieur, avec des moyens de fixation à un corps vertébral inférieur, et, entre les paliers de glissement supérieur et inférieur, un palier de glissement additionnel, intermédiaire, est agencé avec un côté inférieur et un côté supérieur, respectivement des surfaces d'articulation étant situées entre le côté interne du palier de glissement supérieur et le côté supérieur du palier de glissement intermédiaire, de même qu'entre le côté inférieur du palier de glissement intermédiaire et le côté interne du palier de glissement inférieur, dans laquelle
• le palier de glissement intermédiaire (13) comprend des convexités (16) sur les côtés supérieur et inférieur, et la géométrie de ces convexités (16) correspond respectivement à une calotte, et
• les paliers de glissement supérieur et inférieur (11, 12) sont construits avec une concavité (17) sur le côté interne, et la géométrie de ces concavités (17) est définie par des évidements correspondant aux convexités (16) des côtés inférieur et supérieur du palier de glissement intermédiaire (13), les concavités (17) étant respectivement complètement entourées par une bordure (18), et
• soit pour le palier de glissement supérieur soit pour le palier de glissement inférieur (11, 12), la bordure (18) entoure la concavité (17) de manière ondulée, et est ainsi formée pour une limitation prédéfinie du mouvement maximal possible des paliers de glissement (11, 12, 13) l'un vers l'autre en raison de sa hauteur variable, et
• la bordure (18) de l'autre palier de glissement respectif (11, 12), inférieur ou supérieur, entoure la concavité (17) de manière ondulée ou plane,
• dans laquelle les différences de hauteur d'une bordure sous forme ondulée (18) passent de manière fluide les unes dans les autres et/ou en une ou plusieurs marches, et
• pour une inclinaison terminale et/ou une rotation des paliers de glissement (11, 12) l'un vers l'autre, une limitation définie du mouvement maximal possible a lieu suite à une fermeture d'espace des bordures (18), et
**caractérisé en ce que**
• grâce à l'agencement sous forme ondulée d'au moins une bordure (18) du palier de glissement supérieur ou inférieur (11, 12), le mouvement maximal possible des paliers de glissement (11, 12 et 13) l'un vers l'autre est toujours supérieur en direction dorsoventrale par rapport à en direction latéro-latéral.

3. Endoprothèse de disque selon la revendication 2, dans laquelle, pour le palier de glissement intermédiaire (13), les convexités (16) sur les côtés supérieur et inférieur sont chacune entourées par une bordure (18), chacune étant respectivement agencée dans sa largeur totale de sorte d'être de même hauteur sur tout le pourtour ou bien de hauteur différente ou bien sous forme ondulée, les différences de hauteur d'une bordure sous forme ondulée (18) passent de manière fluide les unes dans les autres et/ou en une ou plusieurs marches..

4. Endoprothèse de disque selon au moins l'une des revendications précédentes, dans laquelle grâce à l'agencement de bordure sous forme ondulée d'au moins une bordure (18), une inclinaison plus importante des paliers de glissement (11, 12 et 13) l'un vers l'autre est possible en direction ventrale par rapport à en direction dorsale.

5. Endoprothèse de disque selon au moins l'une des revendications précédentes, dans laquelle l'angle d'inclinaison maximal possible (angle d'ouverture) des paliers de glissement (11, 12 et 13) l'un vers l'autre dépend de :
a) la hauteur de la/des convexité(s) sphérique(s) (16) pour une bordure existante par rapport à la bordure qui l'entoure (18), et
b) des hauteur, inclinaison, et agencement sous forme ondulée respectifs de la bordure (18) entourant la/les convexité(s) (16) et la/les concavité(s) (17).

6. Endoprothèse de disque selon au moins l'une des revendications précédentes, dans laquelle les paliers de glissement (11, 12 et 13) sont conçus en une seule pièce.

7. Endoprothèse de disque selon au moins l'une quelconque des revendications 1 à 5, dans laquelle au moins une palier de glissement (11, 12 et 13) comprend au moins deux parties reliées l'une à l'autre fixement ou fixement mais de manière réversible, dans laquelle soit la/les convexité(s) (16) et/ou concavité(s) (17) forment la pièce, qui est reliée fixement ou fixement mais de manière réversible, au palier de glissement correspondant (11, 12 et 13), soit la/les convexité(s) (16) et/ou la/les concavité(s) (17) comportent à la base des moyens adaptés pour une liaison fixe ou fixe mais réversible.

8. Endoprothèse selon au moins l'une des revendications précédentes, dans laquelle les paliers de glissement (11, 12 et 13) et/ou des parties reliées les unes aux autres comprennent des matériaux identiques ou différents.

9. Endoprothèse de disque selon au moins l'une des revendications précédentes, dans laquelle les surfaces des paliers de glissement (11, 12 et 13) et/ou des parties reliées les unes aux autres sont recouvertes de manière identique ou différente.

10. Endoprothèse de disque selon au moins l'une quelconque des revendications 7 à 9, dans laquelle une liaison fixe ou fixe mais réversible est obtenue par une liaison rainure / ressort, un rail de guidage et un évidement correspondant, un mécanisme de cliquet, un collage ou un vissage.

11. Endoprothèse de disque selon au moins l'une quelconque des revendications 2 à 10, dans laquelle les paliers de glissement supérieur et inférieur (11, 12) comprennent le même matériau ou sont recouverts de manière identique, et le palier de glissement intermédiaire (13) est fabriqué dans un autre matériau ou est recouvert de manière différente.

12. Endoprothèse de disque selon la revendication 2 ou selon au moins l'une des revendications précédentes s'y rapportant, dans laquelle les convexités (16) en forme de calotte sur le côté supérieur et inferieur du palier de glissement intermédiaire (13) ainsi que les concavités correspondantes (17) des paliers de glissement supérieur et inferieur (11, 12) comprennent des rayons identiques ou différents.

13. Endoprothèse de disque selon la revendication 2 ou selon l'une des revendications s'y rapportant, dans laquelle la hauteur maximale des convexités (16) du palier de glissement intermédiaire (13) est identique ou différente sur le côté supérieur et inférieur.

14. Endoprothèse de disque selon au moins l'une des revendications précédentes, dans laquelle la hauteur maximale d'une convexité (16) est inférieure à une hémisphère et/ou pour une bordure existante la hauteur de la bordure (18) est réduite, dans laquelle, pour une endoprothèse de disque en trois parties, la hauteur des convexités (16) est identique ou différente sur le côté supérieur et le côté inférieur.

15. Endoprothèse de disque selon au moins l'une des revendications précédentes, dans laquelle l'angle d'ouverture maximum pour une fermeture d'espace des paliers de glissement (11, 12 et 13) d'un côté pendant l'extension ou la flexion s'élève entre 6 et 10 degrés, et pour une fermeture d'espace latéral entre 0 et 6 degrés, avec une tolérance de 3 degrés supplémentaires dans chaque direction.

16. Endoprothèse de disque selon au moins l'une des revendications précédentes, dans laquelle l'agencement des bordures (18) rend possible un mouvement de rotation limité des paliers de glissement (11, 12 et 13) par rapport à un axe vertical central virtuel, jusqu'à 3° pour la colonne vertébrale et jusqu'à 6° pour la colonne cervicale, de chaque côté, avec une tolérance supplémentaire de 2 degrés supplémentaires dans chaque côté.

17. Endoprothèse de disque selon au moins l'une des revendications précédentes, dans laquelle la/les convexité(s) (16) et la/les concavité (s) correspondantes (17) sont décalées vers la section dorsale de jusqu'à 4mm par rapport à la section sagittale centrale.

18. Endoprothèse de disque selon au moins l'une des revendications précédentes, dans laquelle les bordures (18) des paliers de glissement (11, 12 et 13) se terminent rectangulairement vers l'extérieur, angulairement, de manière courbée ou droite associées, de manière courbée et/ou angulaire.

19. Endoprothèse de disque selon la revendication 2 ou au moins l'une des revendications précédentes s'y rapportant dans laquelle, en tant que sécurité supplémentaire pour un palier de glissement intermédiaire (13) avec bordure (18) contre une sortie hors de la prothèse lors de la fermeture d'espace des trois paliers de glissement (11, 12, 13), une butée fait partie de la bordure (18) du palier de glissement intermédiaire (13), laquelle est disposée en dehors du palier de glissement supérieur et/ou inférieur (11, 12), la butée étant plus haute au moins sur le côté supérieur ou inférieur que la bordure (18) du palier de glissement intermédiaire (13).

20. Endoprothèse de disque selon la revendication 2 ou au moins l'une des revendications précédentes s'y rapportant dans laquelle, en tant que sécurité supplémentaire pour un palier de glissement intermédiaire (13) avec bordure (18) contre une sortie en dehors de la prothèse lors de la fermeture d'espace des trois paliers de glissement (11, 12, 13), une butée fait partie de la bordure (18) du palier de glissement intermédiaire (13), la butée étant plus haute sur le côté supérieur et/ou inférieur que la bordure (18) du palier de glissement intermédiaire (13), et la butée est insérée à l'intérieur d'une rainure dans la zone de bordure du palier de glissement supérieur et/ou inférieur (11, 12) avec le jeu nécessaire pour le mouvement de glissement maximal des paliers de glissement (11, 12, 13).

21. Endoprothèse de disque selon la revendication 2 ou au moins l'une des revendications précédentes s'y rapportant dans laquelle, en tant que sécurité supplémentaire pour un palier de glissement intermédiaire (13) avec bordure (18), contre une sortie en dehors de la prothèse lors de la fermeture d'espace des trois paliers de glissement (11, 12, 13), la bordure (18) du palier de glissement intermédiaire (13) devient plus haute, partiellement ou globalement en continu, à partir de la zone de passage de la convexité vers la périphérie, et la bordure du palier de glissement supérieur et/ou inférieur (11, 12) devient plus plate dans une mesure identique.

22. Endoprothèse de disque selon la revendication 2 ou au moins l'une des revendications précédentes s'y rapportant, dans laquelle, en tant que sécurité supplémentaire pour un palier de glissement intermédiaire (13) contre une sortie en dehors de la prothèse lors de la fermeture d'espace des trois paliers de glissement (11, 12, 13), les contours externes du palier de glissement supérieur et/ou inférieur (11, 12) sont conçus totalement ou partiellement en forme de crochets, rectangulairement, angulairement autrement, de manière courbée, ou en association de cela en direction des autres paliers de glissement externes.

23. Endoprothèse de disque selon au moins l'une des revendications précédentes, dans laquelle une surface et forme de la périphérie externe du palier de glissement supérieur et inférieur (11, 12) sont identiques ou différentes, et sont ainsi adaptées aux dimensions correspondantes du corps vertébral auquel elles sont reliées.

24. Endoprothèse de disque selon au moins l'une des revendications précédentes, dans laquelle le palier de glissement supérieur et/ou le palier de glissement inférieur (11, 12) est/sont conçu(s) de sorte que, en coupe frontale et/sagittale, les côtés extérieur et intérieur du/des palier(s) de glissement supérieur et/ou inférieur (11, 12) sont disposés de manière parallèle ou non parallèle les uns par rapport aux autres.

25. Endoprothèse de disque selon au moins l'une des revendications précédentes, dans laquelle la convexité d'une prothèse en deux parties (11, 12) et les côtés supérieur et inférieur du palier de glissement intermédiaire (13) d'une prothèse en trois parties sont parallèles ou non parallèles par rapport à une horizontale, et se trouvent ainsi dans un angle défini l'un par rapport à l'autre, la/les convexité(s) étant symétrique(s) ou asymétrique(s).

26. Endoprothèse de disque selon au moins l'une des revendications précédentes, dans laquelle les paliers de glissement supérieur et inférieur (11, 12) sont plans ou convexes et recouverts sur le côté extérieur de manière bioactive, ou sont émoussés, et comportent des rangées de petites dents d'ancrage (20) pour leur ancrage primaire avec les corps vertébraux, lesquelles sont disposées de manière droite ou inclinées latéralement ou dans une direction latérale, ventrale et dorsale, et dans le sens dorsal des rangées se trouvant uniquement sur les côtés des petites dents d'ancrage (20).

27. Endoprothèse de disque selon au moins l'une des revendications précédentes, dans laquelle les paliers de glissement supérieur et/ou inférieur (11, 12) comportent des moyens de mise en prise avec un instrument d'implantation ou d'explantation.

28. Endoprothèse de disque selon au moins l'une des revendications précédentes, dans laquelle celle-ci comprend une largeur maximale (vue de face) de 14 à 48 mm, une profondeur maximale (coupe sagittale) de 11 à 35 mm, et une hauteur maximale de 4 à 18 mm.

29. Endoprothèse de disque selon au moins l'une des revendications 1 à 28, pour une implantation dans la colonne vertébrale, dans laquelle la périphérie externe des paliers de glissement supérieur et inférieur (11, 12) se réduit ventralement en vue transversale.

30. Endoprothèse de disque selon au moins l'une des revendications 1 à 28, pour l'implantation dans la colonne vertébrale, dans laquelle la périphérie externe des paliers de glissement supérieur et inférieur (11, 12) se réduit vers le dos en vue transversale.

31. Endoprothése de disque selon la revendication 29 ou 30, dans laquelle le rétrécissement de la périphérie externe des paliers de glissement (11, 12) est conçu latéralement comme une courbure identique ou asymétrique.

32. Endoprothèse de disque selon au moins l'une des revendications précédentes, dans laquelle la prothèse comprend respectivement sous la surface un ou plusieurs marquages visibles au rayon X.
